# EUROPEAN PATENT APPLICATION

(11) **EP 4 424 827 A1**
(43) Date of publication of application: **04.09.2024**
(21) Application number: 22887073.9
(22) Date of filing: 26.10.2022
(51) Int. Cl.: C12N 15/113, A61K 31/7088, A61K 48/00, A61P 31/14

(54) **ANTIVIRAL ANTISENSE OLIGONUCLEOTIDE**

(30) Priority: 26.10.2021 JP 2021174879
(71) Applicant: Nippon Shinyaku Co., Ltd., Kyoto-shi Kyoto 601-8550 (JP); National University Corporation Gunma University, Maebashi-shi, Gunma 371-8510 (JP)
(72) Inventor: NAKAGAWA, Shinichiro, Tsukuba-shi, Ibaraki 305-0003 (JP); TAGAYA, Mitsuhiro, Tsukuba-shi, Ibaraki 305-0003 (JP); HIMOTO, Takuya, Tsukuba-shi, Ibaraki 305-0003 (JP); KIYAMA, Kaname, Tsukuba-shi, Ibaraki 305-0003 (JP); TOJO, Akari, Tsukuba-shi, Ibaraki 305-0003 (JP); KAMITANI, Wataru, Maebashi-shi, Gunma 371-8510 (JP)
(74) Representative: Hoffmann Eitle
(86) International application number: PCT/JP2022/039960
(87) International publication number: WO 2023/074748

(57) **Abstract**

The present specification provides an antisense oligonucleotide or a pharmaceutically acceptable salt or hydrate thereof targeting a particular region in genomic RNA of SARS-CoV-2, a pharmaceutical composition comprising the antisense oligonucleotide or the pharmaceutically acceptable salt or hydrate thereof, etc.

## Description

### Technical Field

The present invention relates to an antisense oligonucleotide or a pharmaceutically acceptable salt or hydrate thereof having an antiviral effect on SARS-CoV-2, SARS-CoV-1, or MERS-CoV (hereinafter, also referred to as the "antisense oligonucleotide, etc."); a pharmaceutical composition comprising the antisense oligonucleotide, etc.; or a method for treatment and/or prevention of viral infection, which comprises administering to a subject the antisense oligonucleotide, etc. or the pharmaceutical composition.

### Background Art

COVID-19 (coronavirus disease-2019) is a novel infection characterized by pneumonia which was first confirmed in Wuhan, Hubei Province, China in November 2019 and then declared a pandemic by WHO in March 2020 (Non Patent Literature 1). The cause of this COVID-19 is a novel virus, and the causative virus was identified as severe acute respiratory syndrome coronavirus 2 (SARS-CoV-2) in January 2020. SARS-CoV-2 is evolutionarily closely related to SARS-CoV, a causative virus of severe acute respiratory syndrome (SARS) spread in 2003, and belongs to the genus *Betacoronavirus,* as in SARS-CoV (Non Patent Literature 2).

### Citation List

### Non Patent Literature

Non Patent Literature 1: World Health Organization (WHO) (Press release). 11 March 2020
Non Patent Literature 2: Nat Microbiol. 2020 Apr; 5 (4): 536-544

### Summary of Invention

### Technical Problem

Although use of existing antiviral agents, for example, has been proposed for SARS-CoV-2, their effects have not yet been clinically demonstrated and there is no established treatment method. Also, there is no established treatment method as to SARS-CoV-1 and MERS-CoV which belong to the genus *Betacoronavirus,* as in SARS-CoV-2, and bring about viral infection.

Under these circumstances, it has been demanded to provide a novel therapeutic agent or the like having an antiviral effect on SARS-CoV-2, SARS-CoV-1, or MERS-CoV.

### Solution to Problem

The present invention provides an antisense oligonucleotide or a pharmaceutically acceptable salt or hydrate thereof targeting a particular region described below in genomic RNA of SARS-CoV-2, a pharmaceutical composition comprising the antisense oligonucleotide or the pharmaceutically acceptable salt or hydrate thereof, etc.

(1) An antisense oligonucleotide consisting of 15 to 30 nucleotides or a pharmaceutically acceptable salt or hydrate thereof, the 15 to 30 nucleotides being complementary to a nucleic acid comprising at least 10 consecutive bases in at least one target region selected from the group consisting of 5' UTR region, nsp1 region, nsp3 region, nsp4 region, 3C-like proteinase region, nsp6 region, nsp7 region, nsp8 region, nsp9 region, nsp10 region, RNA-dependent RNA polymerase region, helicase region, 3'-to-5' exonuclease region, endoRNase region, 2'-O-ribose methyltransferase region, S region comprising S1 region and S2 region, ORF3a region, E region, M region, ORF7b region, N region, ORF10 region, and 3' UTR region in genomic RNA of SARS-CoV-2, wherein
   the antisense oligonucleotide or the pharmaceutically acceptable salt or hydrate thereof has an antiviral effect on a virus selected from the group consisting of SARS-CoV-2, SARS-CoV-1, and MERS-CoV.
(2) The antisense oligonucleotide or the pharmaceutically acceptable salt or hydrate thereof according to (1), wherein the antisense oligonucleotide is a gapmer comprising a center gap region and two wing regions flanking the gap region.
(3) The antisense oligonucleotide or the pharmaceutically acceptable salt or hydrate thereof according to (1) or (2), wherein the virus is SARS-CoV-2 or SARS-CoV-1.
(4) The antisense oligonucleotide or the pharmaceutically acceptable salt or hydrate thereof according to any of (1) to (3), wherein the at least one target region is selected from the group consisting of nsp1 region, nsp3 region, 3C-like proteinase region, nsp8 region, RNA-dependent RNA polymerase region, helicase region, 3'-to-5' exonuclease region, endoRNase region, 2'-O-ribose methyltransferase region, S region comprising S1 region and S2 region, M region, ORF7b region, N region, ORF10 region, and 3' UTR region in genomic RNA of SARS-CoV-2.
(5) The antisense oligonucleotide or the pharmaceutically acceptable salt or hydrate thereof according to any of (1) to (4), wherein the at least one target region is selected from the group consisting of nsp3 region, RNA-dependent RNA polymerase region, helicase region, 3'-to-5' exonuclease region, endoRNase region, 2'-O-ribose methyltransferase region, S region comprising S1 region and S2 region, and N region in genomic RNA of SARS-CoV-2.
(6) The antisense oligonucleotide or the pharmaceutically acceptable salt or hydrate thereof according to any of (1) to (5), wherein the antisense oligonucleotide is complementary to a nucleic acid comprising at least 10 consecutive bases in at least one target region selected from the group consisting of positions 43 to 89, positions 242 to 279, positions 290 to 312, positions 455 to 477, positions 704 to 723, positions 3352 to 3372, positions 5384 to 5403, positions 6071 to 6090, positions 6406 to 6427, positions 6797 to 6815, positions 7532 to 7551, positions 8707 to 8724, positions 10292 to 10309, positions 10406 to 10431, positions 10484 to 10506, positions 11609 to 11630, positions 12023 to 12045, positions 12170 to 12188, positions 12212 to 12234, positions 12314 to 12339, positions 12401 to 12420, positions 12589 to 12608, positions 12839 to 12867, positions 12898 to 12922, positions 12965 to 12990, positions 13151 to 13175, positions 13271 to 13290, positions 13363 to 13386, positions 13458 to 13502, positions 13642 to 13661, positions 13672 to 13691, positions 13762 to 13790, positions 13894 to 13916, positions 14050 to 14069, positions 14290 to 14312, positions 14512 to 14531, positions 14654 to 14687, positions 14698 to 14717, positions 14750 to 14777, positions 14824 to 14846, positions 14854 to 14873, positions 14878 to 14909, positions 14953 to 14990, positions 14992 to 15026, positions 15037 to 15061, positions 15063 to 15140, positions 15172 to 15198, positions 15278 to 15299, positions 15454 to 15479, positions 15496 to 15518, positions 15520 to 15539, positions 15622 to 15644, positions 15791 to 15809, positions 15829 to 15859, positions 15886 to 15905, positions 15929 to 15950, positions 15985 to 16011, positions 16051 to 16085, positions 16189 to 16220, positions 16430 to 16451, positions 16636 to 16655, positions 16822 to 16845, positions 17015 to 17051, positions 17080 to 17103, positions 17137 to 17156, positions 17215 to 17234, positions 17254 to 17277, positions 17564 to 17600, positions 17748 to 17765, positions 17773 to 17792, positions 17830 to 17849, positions 17859 to 17876, positions 18094 to 18111, positions 18196 to 18218, positions 18253 to 18278, positions 18370 to 18387, positions 19568 to 19595, positions 19622 to 19639, positions 19780 to 19802, positions 20107 to 20130, positions 20776 to 20795, positions 20797 to 20816, positions 20888 to 20909, positions 21453 to 21472, positions 21502 to 21524, positions 22550 to 22569, positions 22814 to 22836, positions 23093 to 23113, positions 23956 to 23976, positions 24302 to 24324, positions 24446 to 24465, positions 24467 to 24489, positions 24620 to 24651, positions 24662 to 24684, positions 24962 to 24982, positions 25104 to 25128, positions 25364 to 25384, positions 25502 to 25520, positions 26287 to 26325, positions 26574 to 26604, positions 26782 to 26800, positions 27093 to 27111, positions 27771 to 27794, positions 27806 to 27823, positions 28270 to 28294, positions 28397 to 28418, positions 28509 to 28538, positions 28744 to 28784, positions 28799 to 28820, positions 28946 to 28972, positions 28986 to 29005, positions 29010 to 29031, positions 29102 to 29130, positions 29174 to 29196, positions 29354 to 29373, positions 29615 to 29634, positions 29712 to 29731, and positions 29787 to 29867 of SEQ ID NO: 1.
(7) The antisense oligonucleotide or the pharmaceutically acceptable salt or hydrate thereof according to any of (1) to (6), wherein the antisense oligonucleotide is complementary to a nucleic acid comprising at least 10 consecutive bases in at least one target region selected from the group consisting of positions 47 to 66, positions 242 to 261, positions 259 to 278, positions 292 to 311, positions 456 to 475, positions 704 to 723, positions 3353 to 3372, positions 5384 to 5403, positions 6071 to 6090, positions 6406 to 6425, positions 6407 to 6426, positions 6408 to 6427, positions 6797 to 6815, positions 7532 to 7551, positions 8707 to 8724, positions 10292 to 10309, positions 10407 to 10426, positions 10409 to 10428, positions 10487 to 10506, positions 11609 to 11628, positions 12025 to 12044, positions 12170 to 12188, positions 12214 to 12233, positions 12316 to 12335, positions 12401 to 12420, positions 12589 to 12608, positions 12843 to 12862, positions 12900 to 12919, positions 12969 to 12988, positions 13153 to 13172, positions 13271 to 13290, positions 13366 to 13385, positions 13462 to 13481, positions 13481 to 13500, positions 13642 to 13661, positions 13672 to 13691, positions 13767 to 13786, positions 13894 to 13913, positions 14050 to 14069, positions 14292 to 14311, positions 14512 to 14531, positions 14656 to 14675, positions 14668 to 14687, positions 14698 to 14717, positions 14753 to 14772, positions 14826 to 14845, positions 14854 to 14873, positions 14879 to 14898, positions 14889 to 14908, positions 14962 to 14981, positions 14971 to 14990, positions 14992 to 15011, positions 15007 to 15026, positions 15040 to 15059, positions 15064 to 15083, positions 15091 to 15107, positions 15092 to 15111, positions 15120 to 15139, positions 15172 to 15191, positions 15173 to 15192, positions 15174 to 15193, positions 15175 to 15194, positions 15179 to 15198, positions 15280 to 15299, positions 15280 to 15296, positions 15457 to 15476, positions 15496 to 15515, positions 15497 to 15516, positions 15498 to 15517, positions 15499 to 15518, positions 15520 to 15539, positions 15623 to 15642, positions 15791 to 15809, positions 15834 to 15853, positions 15886 to 15905, positions 15931 to 15950, positions 15988 to 16007, positions 16052 to 16071, positions 16066 to 16085, positions 16189 to 16208, positions 16192 to 16211, positions 16193 to 16212, positions 16194 to 16213, positions 16195 to 16214, positions 16196 to 16215, positions 16198 to 16217, positions 16201 to 16220, positions 16432 to 16451, positions 16636 to 16655, positions 16825 to 16844, positions 17016 to 17035, positions 17032 to 17051, positions 17080 to 17099, positions 17083 to 17102, positions 17084 to 17103, positions 17137 to 17156, positions 17215 to 17234, positions 17257 to 17276, positions 17564 to 17583, positions 17565 to 17584, positions 17566 to 17585, positions 17567 to 17586, positions 17569 to 17588, positions 17573 to 17592, positions 17576 to 17595, positions 17580 to 17599, positions 17748 to 17765, positions 17773 to 17792, positions 17830 to 17849, positions 17859 to 17876, positions 18094 to 18111, positions 18197 to 18216, positions 18257 to 18276, positions 18370 to 18387, positions 19572 to 19591, positions 19622 to 19639, positions 19780 to 19799, positions 19783 to 19802, positions 20107 to 20126, positions 20108 to 20127, positions 20109 to 20128, positions 20110 to 20129, positions 20111 to 20130, positions 20776 to 20795, positions 20797 to 20816, positions 20889 to 20908, positions 21453 to 21472, positions 21502 to 21521, positions 21503 to 21522, positions 21504 to 21523, positions 22550 to 22569, positions 22817 to 22836, positions 23093 to 23112, positions 23957 to 23976, positions 24303 to 24322, positions 24446 to 24465, positions 24469 to 24488, positions 24620 to 24639, positions 24632 to 24651, positions 24665 to 24684, positions 24962 to 24981, positions 25107 to 25126, positions 25365 to 25384, positions 25502 to 25520, positions 26287 to 26306, positions 26305 to 26324, positions 26579 to 26598, positions 26782 to 26800, positions 27093 to 27111, positions 27774 to 27793, positions 27806 to 27823, positions 28274 to 28293, positions 28398 to 28417, positions 28514 to 28533, positions 28744 to 28763, positions 28745 to 28764, positions 28746 to 28765, positions 28747 to 28766, positions 28748 to 28767, positions 28752 to 28771, positions 28756 to 28775, positions 28757 to 28776, positions 28758 to 28777, positions 28759 to 28778, positions 28762 to 28781, positions 28765 to 28784, positions 28799 to 28818, positions 28950 to 28969, positions 28986 to 29005, positions 29010 to 29029, positions 29106 to 29125, positions 29176 to 29195, positions 29354 to 29373, positions 29615 to 29634, positions 29712 to 29731, and positions 29789 to 29808 of SEQ ID NO: 1.
(8) The antisense oligonucleotide or the pharmaceutically acceptable salt or hydrate thereof according to any of (1) to (7), wherein the antisense oligonucleotide is complementary to a nucleic acid comprising at least 10 consecutive bases in at least one target region selected from the group consisting of positions 704 to 723, positions 5384 to 5403, positions 6407 to 6426, positions 10409 to 10428, positions 10487 to 10506, positions 12170 to 12188, positions 12214 to 12233, positions 12316 to 12335, positions 12401 to 12420, positions 13642 to 13661, positions 13672 to 13691, positions 13767 to 13786, positions 13894 to 13913, positions 14050 to 14069, positions 14512 to 14531, positions 14656 to 14675, positions 14668 to 14687, positions 14753 to 14772, positions 14826 to 14845, positions 14879 to 14898, positions 14889 to 14908, positions 14962 to 14981, positions 14992 to 15011, positions 15007 to 15026, positions 15040 to 15059, positions 15064 to 15083, positions 15091 to 15107, positions 15092 to 15111, positions 15120 to 15139, positions 15173 to 15192, positions 15175 to 15194, positions 15179 to 15198, positions 15280 to 15299, positions 15280 to 15296, positions 15457 to 15476, positions 15496 to 15515, positions 15498 to 15517, positions 15791 to 15809, positions 15834 to 15853, positions 15886 to 15905, positions 15931 to 15950, positions 15988 to 16007, positions 16052 to 16071, positions 16066 to 16085, positions 16189 to 16208, positions 16192 to 16211, positions 16195 to 16214, positions 16201 to 16220, positions 16432 to 16451, positions 16636 to 16655, positions 17016 to 17035, positions 17032 to 17051, positions 17083 to 17102, positions 17084 to 17103, positions 17257 to 17276, positions 17565 to 17584, positions 17576 to 17595, positions 17580 to 17599, positions 17748 to 17765, positions 17773 to 17792, positions 17830 to 17849, positions 18094 to 18111, positions 18197 to 18216, positions 18257 to 18276, positions 19622 to 19639, positions 19783 to 19802, positions 20108 to 20127, positions 20111 to 20130, positions 20776 to 20795, positions 20797 to 20816, positions 20889 to 20908, positions 21502 to 21521, positions 21504 to 21523, positions 24469 to 24488, positions 24620 to 24639, positions 24632 to 24651, positions 24665 to 24684, positions 24962 to 24981, positions 25107 to 25126, positions 26782 to 26800, positions 27806 to 27823, positions 28274 to 28293, positions 28514 to 28533, positions 28745 to 28764, positions 28746 to 28765, positions 28756 to 28775, positions 28757 to 28776, positions 28758 to 28777, positions 28759 to 28778, positions 28765 to 28784, positions 28799 to 28818, positions 28950 to 28969, positions 29010 to 29029, positions 29106 to 29125, positions 29176 to 29195, positions 29354 to 29373, positions 29615 to 29634, and positions 29712 to 29731 of SEQ ID NO: 1.
(9) The antisense oligonucleotide or the pharmaceutically acceptable salt or hydrate thereof according to any of (1) to (8), wherein the antisense oligonucleotide is complementary to a nucleic acid comprising at least 10 consecutive bases in at least one target region selected from the group consisting of positions 5384 to 5403, positions 6407 to 6426, positions 13642 to 13661, positions 13672 to 13691, positions 15173 to 15192, positions 15175 to 15194, positions 15179 to 15198, positions 15496 to 15515, positions 15498 to 15517, positions 16189 to 16208, positions 16192 to 16211, positions 16195 to 16214, positions 16201 to 16220, positions 17083 to 17102, positions 17084 to 17103, positions 17565 to 17584, positions 17576 to 17595, positions 18257 to 18276, positions 19783 to 19802, positions 20108 to 20127, positions 20111 to 20130, positions 21502 to 21521, positions 21504 to 21523, positions 24665 to 24684, positions 28745 to 28764, positions 28746 to 28765, positions 28756 to 28775, positions 28757 to 28776, positions 28758 to 28777, and positions 28759 to 28778 of SEQ ID NO: 1.
(10) The antisense oligonucleotide or the pharmaceutically acceptable salt or hydrate thereof according to any of (1) to (9), wherein the antisense oligonucleotide is complementary to a nucleic acid comprising at least 10 consecutive bases in at least one target region selected from the group consisting of positions 6407 to 6426, positions 15173 to 15192, positions 15496 to 15515, positions 15498 to 15517, positions 16195 to 16214, positions 17084 to 17103, positions 20111 to 20130, positions 21502 to 21521, positions 28757 to 28776, and positions 28758 to 28777 of SEQ ID NO: 1.
(11) The antisense oligonucleotide or the pharmaceutically acceptable salt or hydrate thereof according to any of (1) to (10), wherein the antisense oligonucleotide is complementary to a nucleic acid comprising at least 15 consecutive bases in the target region.
(12) The antisense oligonucleotide or the pharmaceutically acceptable salt or hydrate thereof according to any of (1) to (11), wherein the antisense oligonucleotide comprises
   (a) a base sequence selected from the group consisting of SEQ ID NOs: 2 to 177,
   (b) a base sequence having addition, deletion, or substitution of one or several base(s) in the base sequence selected from the group consisting of SEQ ID NOs: 2 to 177, or
   (c) a base sequence having at least 80% sequence identity to the base sequence selected from the group consisting of SEQ ID NOs: 2 to 177, and
   inhibits a function of the target region.
(13) The antisense oligonucleotide or the pharmaceutically acceptable salt or hydrate thereof according to any of (1) to (12), wherein the antisense oligonucleotide comprises
   (a) a base sequence selected from the group consisting of SEQ ID NOs: 7, 9, 12, 19, 20, 23 to 26, 36 to 40, 42 to 44, 46, 47, 49 to 51, 53 to 59, 61, 63 to 68, 70, 74 to 82, 85, 88 to 90, 92, 93, 95, 96, 99, 101, 106 to 110, 112 to 114, 117, 119, 121, 124, 125 to 127, 129, 131, 138 to 143, 149, 152, 153, 155, 157, 158, 162 to 165, 167 to 169, and 171 to 176,
   (b) a base sequence having addition, deletion, or substitution of one or several base(s) in the base sequence selected from the group consisting of SEQ ID NOs: 7, 9, 12, 19, 20, 23 to 26, 36 to 40, 42 to 44, 46, 47, 49 to 51, 53 to 59, 61, 63 to 68, 70, 74 to 82, 85, 88 to 90, 92, 93, 95, 96, 99, 101, 106 to 110, 112 to 114, 117, 119, 121, 124, 125 to 127, 129, 131, 138 to 143, 149, 152, 153, 155, 157, 158, 162 to 165, 167 to 169, and 171 to 176, or
   (c) a base sequence having at least 80% sequence identity to the base sequence selected from the group consisting of SEQ ID NOs: 7, 9, 12, 19, 20, 23 to 26, 36 to 40, 42 to 44, 46, 47, 49 to 51, 53 to 59, 61, 63 to 68, 70, 74 to 82, 85, 88 to 90, 92, 93, 95, 96, 99, 101, 106 to 110, 112 to 114, 117, 119, 121, 124, 125 to 127, 129, 131, 138 to 143, 149, 152, 153, 155, 157, 158, 162 to 165, 167 to 169, and 171 to 176, and
   inhibits a function of the target region.
(14) The antisense oligonucleotide or the pharmaceutically acceptable salt or hydrate thereof according to any of (1) to (13), wherein the antisense oligonucleotide comprises
   (a) a base sequence selected from the group consisting of SEQ ID NOs: 9, 12, 36, 37, 61, 63, 64, 68, 70, 81, 82, 85, 88, 95, 96, 101, 106, 114, 119, 121, 124, 129, 131, 141, 157, 158, 162, 163, 164, and 165,
   (b) a base sequence having addition, deletion, or substitution of one or several base(s) in the base sequence selected from the group consisting of SEQ ID NOs: 9, 12, 36, 37, 61, 63, 64, 68, 70, 81, 82, 85, 88, 95, 96, 101, 106, 114, 119, 121, 124, 129, 131, 141, 157, 158, 162, 163, 164, and 165, or
   (c) a base sequence having at least 80% sequence identity to the base sequence selected from the group consisting of SEQ ID NOs: 9, 12, 36, 37, 61, 63, 64, 68, 70, 81, 82, 85, 88, 95, 96, 101, 106, 114, 119, 121, 124, 129, 131, 141, 157, 158, 162, 163, 164, and 165, and
   inhibits a function of the target region.
(15) The antisense oligonucleotide or the pharmaceutically acceptable salt or hydrate thereof according to any of (1) to (14), wherein the antisense oligonucleotide comprises
   (a) a base sequence selected from the group consisting of SEQ ID NOs: 12, 61, 68, 70, 85, 96, 124, 129, 163, and 164,
   (b) a base sequence having addition, deletion, or substitution of one or several base(s) in the base sequence selected from the group consisting of SEQ ID NOs: 12, 61, 68, 70, 85, 96, 124, 129, 163, and 164, or
   (c) a base sequence having at least 80% sequence identity to the base sequence selected from the group consisting of SEQ ID NOs: 12, 61, 68, 70, 85, 96, 124, 129, 163, and 164, and
   inhibits a function of the target region.
(16) The antisense oligonucleotide or the pharmaceutically acceptable salt or hydrate thereof according to any of (11) to (15), wherein the antisense oligonucleotide comprises the sequence (a).
(17-1) The antisense oligonucleotide or the pharmaceutically acceptable salt or hydrate thereof according to any of (1) to (16), wherein the antisense oligonucleotide consists of 20 nucleotides.
(17-2) The antisense oligonucleotide or the pharmaceutically acceptable salt or hydrate thereof according to (17-1), wherein the antisense oligonucleotide is a gapmer constituted by a wing region of 5 nucleotides long, a gap region of 10 nucleotides long, and a wing region of 10 nucleotides long from the 5' end toward the 3' end.
(18) The antisense oligonucleotide or the pharmaceutically acceptable salt or hydrate thereof according to any of (1) to (17-2), wherein the wing regions comprise a 2'-OMe (2'-O-CH₃) group and/or a 2'-O-MOE (2'-O-CH₂CH₂OCH₃) group.
(19) The antisense oligonucleotide or the pharmaceutically acceptable salt or hydrate thereof according to (17-1), (17-2), or (18), wherein a bond between the second and third nucleosides and a bond between the fourth and fifth nucleosides from the 5' end of the 5' wing region are phosphodiester bonds, and a bond between the first and second nucleosides and a bond between the third and fourth nucleosides from the 5' end of the 3' wing region are phosphodiester bonds, while all bonds between the other nucleosides are phosphorothioate bonds.
(20) The antisense oligonucleotide or the pharmaceutically acceptable salt or hydrate thereof according to (17-1), (17-2), or (18), wherein a bond between the second and third nucleosides, a bond between the third and fourth nucleosides, and a bond between the fourth and fifth nucleosides from the 5' end of the 5' wing region are phosphodiester bonds, and a bond between the first and second nucleosides, a bond between the second and third nucleosides, and a bond between the third and fourth nucleosides from the 5' end of the 3' wing region are phosphodiester bonds, while all bonds between the other nucleosides are phosphorothioate bonds.
(21) A pharmaceutical composition comprising the antisense oligonucleotide or the pharmaceutically acceptable salt or hydrate thereof according to any of (1) to (20).
(22) The pharmaceutical composition according to (21) for the treatment and/or prevention of infection with a virus selected from the group consisting of SARS-CoV-2, SARS-CoV-1, and MERS-CoV.
(23) A method for treatment and/or prevention of infection with a virus selected from the group consisting of SARS-CoV-2, SARS-CoV-1, and MERS-CoV, which comprises administering to a subject an effective amount of the antisense oligonucleotide or the pharmaceutically acceptable salt or hydrate thereof according to any of (1) to (20) or the pharmaceutical composition according to (21) or (22).

### Advantageous Effects of Invention

The present invention provides an antisense oligonucleotide or a pharmaceutically acceptable salt or hydrate thereof targeting genomic RNA of SARS-CoV-2, and a composition comprising the antisense oligonucleotide or the pharmaceutically acceptable salt or hydrate thereof, etc.

According to a preferred aspect of the present invention, the antisense oligonucleotide of the present invention is capable of providing a therapeutic agent and/or a prophylactic agent for viruses having a high viral growth inhibitory effect and/or few adverse reactions. According to a preferred aspect of the present invention, the antisense oligonucleotide of the present invention is capable of exerting an effect on known SARS-related coronavirus (SARSr-CoV) such as a mutant of SARS-CoV-2 and/or SARS-CoV-1 and/or unknown SARS-related coronavirus.

### Description of Embodiments

In one embodiment, the present invention relates to an antisense oligonucleotide or a pharmaceutically acceptable salt or hydrate thereof having the antiviral effect against SARS-CoV-2, SARS-CoV-1, or MERS-CoV (hereinafter, the antisense oligonucleotide of the present invention or the pharmaceutically acceptable salt or hydrate thereof is also collectively referred to as the "antisense oligonucleotide of the present invention").

In one embodiment, the antisense oligonucleotide of the present invention consists of 15 to 30 nucleotides complementary to a nucleic acid comprising at least 10 consecutive bases in a target region.

In one embodiment, the antisense oligonucleotide of the present invention is complementary to a nucleic acid comprising at least 10, for example, at least 11, at least 12, at least 13, at least 14, at least 15, at least 16, at least 17, at least 18, at least 19, or at least 20, for example, 20 consecutive bases in a target region, or consisting of these bases.

As used herein, the antisense oligonucleotide "complementary" to a nucleic acid is not limited to an antisense oligonucleotide that forms a Watson-Crick base pair with the target nucleic acid and also comprises an antisense oligonucleotide that forms a Wobble base pair therewith. Herein, the Watson-Crick base pair means a base pair that forms a hydrogen bond between adenine and thymine, between adenine and uracil, or between guanine and cytosine, and the Wobble base pair means a base pair that forms a hydrogen bond between guanine and uracil, between inosine and uracil, between inosine and adenine, or between inosine and cytosine. The term "complementary base sequence" may not have 100% complementarity to the target base sequence and may comprise 1 base, 2 bases, 3 bases, 4 bases, or 5 bases noncomplementary to the target base sequence, and may be a base sequence shorter by 1 base, 2 bases, 3 bases, 4 bases, or 5 bases than the target base sequence. In one embodiment, the antisense oligonucleotide "complementary" to a nucleic acid has at least 90%, 91%, 92%, 93%, 94%, 95%, 96%, 97%, 98%, 99%, or 100% complementarity to the nucleic acid. The complementarity can be readily determined by those skilled in the art and can be calculated, for example, by aligning two sequences, counting the number of bases that form a Watson-Crick base pair or a Wobble base pair between the sequences, dividing the number of bases that have formed a base pair by the total number of bases in the sequences, and multiplying the obtained value by 100.

Examples of the antisense oligonucleotide "complementary" to a nucleic acid include an antisense oligonucleotide capable of hybridizing under stringent conditions to the nucleic acid. As used herein, the term "stringent conditions" may be any of low stringent conditions, moderate stringent conditions or high stringent conditions. The term "low stringent condition" is, for example, 5 × SSC, 5 × Denhardt's solution, 0.5% SDS, 50% formamide at 32°C. The term "moderate stringent condition" is, for example, 5 × SSC, 5 × Denhardt's solution, 0.5% SDS, 50% formamide at 42°C, or 5 × SSC, 1% SDS, 50 mM Tris-HCl (pH 7.5), 50% formamide at 42°C. The term "high stringent condition" is, for example, 5 × SSC, 5 × Denhardt's solution, 0.5% SDS, 50% formamide at 50°C or 0.2 × SSC, 0.1% SDS at 65°C. Under these conditions, base sequences with higher sequence identity are expected to be obtained efficiently at higher temperatures. Multiple factors are involved in hybridization stringency including temperature, probe concentration, probe length, ionic strength, time, salt concentration and others, and those skilled in the art may appropriately select these factors to achieve similar stringency.

When commercially available kits are used for hybridization, for example, an AlkPhos Direct Labelling and Detection System (GE Healthcare) may be used. In this case, according to the attached protocol to the kit, after incubation with a labeled probe overnight, the membrane is washed with a primary wash buffer comprising 0.1% (w/v) SDS at 55°C, thereby enabling to detect hybridization. Alternatively, when the probe is labeled with digoxigenin (DIG) using a commercially available reagent (e.g., a PCR Labelling Mix (Roche Diagnostics), etc.) in producing a probe based on a target sequence, hybridization can be detected with a DIG Nucleic Acid Detection Kit (Roche Diagnostics).

The identity between base sequences can be determined using algorithm BLAST (Basic Local Alignment Search Tool) by Karlin and Altschul (Proc. Natl. Acad. Sci. USA 872264-2268, 1990; and Proc Natl Acad Sci USA 90: 5873, 1993). Programs called BLASTN and BLASTX based on the BLAST algorithm have been developed (Altschul SF, et al: J. Mol. Biol. 215: 403, 1990). When a base sequence is analyzed using BLASTN, the parameters are, for example, score = 100 and wordlength = 12. When BLAST and Gapped BLAST programs are used, the default parameters for each program are employed.

In one embodiment, the antisense oligonucleotide of the present invention can be, for example, 15 or more nucleotides long, 16 or more nucleotides long, 17 or more nucleotides long, 18 or more nucleotides long, 19 or more nucleotides long, 20 or more nucleotides long, 21 or more nucleotides long, 22 or more nucleotides long, 23 or more nucleotides long, 24 or more nucleotides long, 25 or more nucleotides long, 26 or more nucleotides long, 27 or more nucleotides long, 28 or more nucleotides long, 29 or more nucleotides long, or 30 nucleotides long and can be 30 or less nucleotides long, 29 or less nucleotides long, 28 or less nucleotides long, 27 or less nucleotides long, 26 or less nucleotides long, 25 or less nucleotides long, 24 or less nucleotides long, 23 or less nucleotides long, 22 or less nucleotides long, 21 or less nucleotides long, 20 or less nucleotides long, 19 or less nucleotides long, 18 or less nucleotides long, 17 or less nucleotides long, 16 or less nucleotides long, or 15 nucleotides long. The antisense oligonucleotide of the present invention may consist of, for example, 15 to 30 nucleotides, 15 to 25 nucleotides, 16 to 24 nucleotides, 17 to 23 nucleotides, 18 to 22 nucleotides, or 19 to 21 nucleotides, for example, 20 nucleotides.

Examples of the pharmaceutically acceptable salt of the antisense oligonucleotide of the present invention include alkali metal salts such as sodium salt, potassium salt, and lithium salt; alkaline earth metal salts such as calcium salt and magnesium salt; metal salts such as aluminum salt, iron salt, zinc salt, copper salt, nickel salt, cobalt salt, etc.; ammonium salts; organic amine salts such as t-octylamine salt, dibenzylamine salt, morpholine salt, glucosamine salt, phenylglycine alkyl ester salt, ethylenediamine salt, N-methylglucamine salt, guanidine salt, diethylamine salt, triethylamine salt, dicyclohexylamine salt, N,N' -dibenzylethylenediamine salt, chloroprocaine salt, procaine salt, diethanolamine salt, N-benzylphenethylamine salt, piperazine salt, tetramethylammonium salt, tris(hydroxymethyl)aminomethane salt; hydrohalide salts such as hydrofluoric acid salt, hydrochloride salt, hydrobromide salt, and hydroiodide salt; inorganic acid salts such as nitrate salt, perchlorate salt, sulfate salt, phosphate salt, etc.; lower alkane sulfonates such as methanesulfonate salt, trifluoromethanesulfonate salt, and ethanesulfonate salt; arylsulfonate salt such as benzenesulfonate salt and p-toluenesulfonate salt; organic acid salts such as acetate salt, malate salt, fumarate salt, succinate salt, citrate salt, tartrate salt, oxalate salt, maleate salt, etc.; and, amino acid salts such as glycine salt, lysine salt, arginine salt, ornithine salt, glutamic acid salt, and aspartic acid salt. These salts may be produced by known methods. Alternatively, the antisense oligonucleotide of the present invention may be in the form of a hydrate thereof.

The antisense oligonucleotide of the present invention is composed of nucleotides as constituent units. The nucleotides may be any of ribonucleotides, deoxyribonucleotides and modified nucleotides.

The modified nucleotide refers to one having fully or partly modified nucleobases, sugar moieties and/or phosphate bond moieties, which constitute the ribonucleotide or deoxyribonucleotide.

The nucleobase includes, for example, adenine, guanine, hypoxanthine, cytosine, thymine, uracil, and modified bases thereof. Examples of such modified bases include, but not limited to, pseudouracil, 3-methyluracil, dihydrouracil, 5-alkylcytosines (e.g., 5-methylcytosine), 5-alkyluracils (e.g., 5-ethyluracil), 5-halouracils (e.g., 5-bromouracil), 6-azapyrimidine, 6-alkylpyrimidines (e.g., 6-methyluracil), 2-thiouracil, 4-thiouracil, 4-acetylcytosine, 5-(carboxyhydroxymethyl) uracil, 5-carboxymethylaminomethyl-2-thiouracil, 5-carboxymethylaminomethyluracil, 1-methyladenine, 1-methylhypoxanthine, 2,2-dimethylguanine, 3-methylcytosine, 2-methyladenine, 2-methylguanine, N6-methyladenine, 7-methylguanine, 5-methoxyaminomethyl-2-thiouracil, 5-methylaminomethyluracil, 5-methylcarbonylmethyluracil, 5-methyloxyuracil, 5-methyl-2-thiouracil, 2-methylthio-N6-isopentenyladenine, uracil-5-oxyacetic acid, 2-thiocytosine, purine, 2,6-diaminopurine, 2-aminopurine, isoguanine, indole, imidazole, xanthine, etc.

As used herein, thymine "T" and uracil "U" are mutually interchangeable, and whether to be "T" or "U" has no essential influence on the activity of the antisense oligonucleotide of the present invention. The base sequence described herein also comprises the case where "T" is "U", and such base sequences are represented by the same SEQ ID NO. As used herein, a sequence comprising a modified base and a sequence comprising no modified base are represented by the same SEQ ID NO. For example, "cytosine" and "methylcytosine" are mutually interchangeable, and a sequence comprising "cytosine" and a sequence comprising "methylcytosine" instead of "cytosine" are represented by the same SEQ ID NO.

Modification of the sugar moiety may include, for example, modifications at the 2'-position of ribose and modifications of the other positions of the sugar. The modification at the 2'-position of ribose includes a modification replacing the 2'-OH of ribose with -OR,-OROR, -R, -R'OR, -SH, -SR, -NH₂, -NHR, -NR₂, -N₃, -CN, -F, -Cl, -Br or -I, for example, -OMe(-O-CH₃) or-Omethoxyethyl (-O-MOE: -O-CH₂CH₂OCH₃), wherein R represents alkyl, cycloalkyl, acryl or aryl, and R' represents an alkylene.

The modification for the other positions of the sugar includes, for example, replacement of O at the 4' position of ribose or deoxyribose with S, bridging between 2' and 4' positions of the sugar, e.g., LNA (locked nucleic acid) or ENA (2'-O,4'-C-ethylene-bridged nucleic acids), but is not limited thereto.

A modification of the phosphate bond moiety includes, for example, a modification of replacing phosphodiester bond with phosphorothioate bond, phosphorodithioate bond, alkyl phosphonate bond, phosphoramidate bond or boranophosphate bond (cf., *e.g.,* Enya et al: Bioorganic & Medicinal Chemistry, 2008, 18, 9154-9160) (cf., *e.g.*, Japan Domestic Re-Publications of PCT Application No. 2006/129594 and Japan Domestic Re-Publications of PCT Application No. 2006/038608).

As used herein, the alkyl includes preferably a straight or branched alkyl having 1 to 6 carbon atoms. Specific examples include methyl, ethyl, *n*-propyl, isopropyl, *n*-butyl, isobutyl, *sec*-butyl, *tert*-butyl, *n-*pentyl, isopentyl, neopentyl, *tert*-pentyl, *n*-hexyl, and isohexyl. The alkyl may optionally be substituted. Examples of such substituents are a halogen, an alkoxy, cyano, and nitro. The alkyl may be substituted with 1 to 3 substituents.

As used herein, the cycloalkyl includes preferably a cycloalkyl having 3 to 12 carbon atoms. Specific examples include cyclopropyl, cyclobutyl, cyclopentyl, cyclohexyl, cycloheptyl, cyclooctyl, cyclodecyl, and cyclododecyl.

As used herein, the halogen includes fluorine, chlorine, bromine, and iodine.

As used herein, the alkoxy includes a straight or branched alkoxy having 1 to 6 carbon atoms such as methoxy, ethoxy, *n*-propoxy, isopropoxy, *n*-butoxy, isobutoxy, *sec*-butoxy, *tert*-butoxy, *n*-pentyloxy, isopentyloxy, *n*-hexyloxy, isohexyloxy, etc. Among others, an alkoxy having 1 to 3 carbon atoms is preferred.

As used herein, the aryl includes preferably an aryl having 6 to 10 carbon atoms. Specific examples include phenyl, α-naphthyl, and β-naphthyl. Among others, phenyl is preferred. The aryl may optionally be substituted. Examples of such substituents are an alkyl, a halogen, an alkoxy, cyano, and nitro. The aryl may be substituted with one to three of such substituents.

As used herein, the alkylene includes preferably a straight or branched alkylene having 1 to 6 carbon atoms. Specific examples include methylene, ethylene, trimethylene, tetramethylene, pentamethylene, hexamethylene, 2-(ethyl) trimethylene, and 1-(methyl) tetramethylene.

As used herein, the acyl includes a straight or branched alkanoyl or aroyl. Examples of the alkanoyl include formyl, acetyl, 2-methylacetyl, 2,2-dimethylacetyl, propionyl, butyryl, isobutyryl, pentanoyl, 2,2-dimethylpropionyl, hexanoyl, etc. Examples of the aroyl include benzoyl, toluoyl, and naphthoyl. The aroyl may optionally be substituted at substitutable positions and may be substituted with an alkyl(s).

In one embodiment, the antisense oligonucleotide of the present invention is a gapmer comprising a center gap region and two wing regions flanking the 5' end and 3' end of the gap region (also referred to as a 5' wing region and a 3' wing region, respectively). The gap region is a region that is recognized by RNase H and is constituted by deoxyribonucleotides having an unmodified sugar moiety. The wing region comprises at least one modified nucleotide and, for example, is wholly constituted by modified nucleotides (*e.g.*, ribonucleotides modified at the 2'-position of ribose). In one embodiment, the nucleosides of the 5' wing region and the 3' wing region each comprise a modification of at least one, for example, two or more, three or more, four or more, or five or more sugar moieties, for example, a 2'-OMe group and/or a 2'-O-MOE group. For example, all the nucleosides of the 5' wing region and the 3' wing region can comprise a 2'-OMe group and/or a 2'-O-MOE group. Also, the nucleosides of the 5' wing region and the 3' wing region may comprise a modification of the base moiety and may comprise, for example, at least one methylcytosine.

The gap region is not limited by its length and can be, for example, 5 to 15, 8 to 12, 9 to 11 or 10 bases long. The 5' wing region and the 3' wing region are not limited by their lengths and can be each independently 2 to 10, 3 to 8, 4 to 6 or 5 bases long. As used herein, the structure of the gapmer may be represented by "a-b-c type". The term "a-b-c type" means that the gapmer is constituted by a wing region of nucleotide length a, a gap region of nucleotide length b, and a wing region of nucleotide length c from the 5' end toward the 3' end. The gapmer can be, for example, a gapmer of 5-10-5 type, 4-10-5 type, 5-10-4 type, 4-10-4 type, 5-9-4 type, or 4-9-4 type.

In one embodiment, the gapmer of the present invention comprises a modification of at least one phosphate bond moiety, for example, a phosphorothioate bond. For example, 1 or more, 2 or more, 3 or more, 4 or more, 5 or more, 10 or more, 15 or more, or all bonds between nucleotides can be phosphorothioate bonds. In one embodiment, the gapmer of the present invention comprises 1 or more, 2 or more, 3 or more, 4 or more, 5 or more, 6 or more, 7 or more, 8 or more, 9 or more, or 10 or more (e.g., 4 to 6) phosphodiester bonds, for example, in the 5' wing region and/or the 3' wing region, while all bonds between the other nucleosides are phosphorothioate bonds. In one embodiment, a bond between the second and third nucleosides and a bond between the fourth and fifth nucleosides from the 5' end of the 5' wing region are phosphodiester bonds, and a bond between the first and second nucleosides and a bond between the third and fourth nucleosides from the 5' end of the 3' wing region are phosphodiester bonds, while all bonds between the other nucleosides are phosphorothioate bonds. In one embodiment, a bond between the second and third nucleosides, a bond between the third and fourth nucleosides, and a bond between the fourth and fifth nucleosides from the 5' end of the 5' wing region are phosphodiester bonds, and a bond between the first and second nucleosides, a bond between the second and third nucleosides, and a bond between the third and fourth nucleosides from the 5' end of the 3' wing region are phosphodiester bonds, while all bonds between the other nucleosides are phosphorothioate bonds. In one embodiment, the gapmer of the present invention comprises no modification of the phosphate bond moiety, and all bonds between nucleotides can be phosphate bonds.

In one embodiment, the antisense oligonucleotide of the present invention has an antiviral effect on SARS-CoV-2, SARS-CoV-1 or MERS-CoV, for example, SARS-CoV-2 or SARS-CoV-1, for example, SARS-CoV-2. As used herein, the SARS-CoV-2 encompasses a virus having a genomic RNA sequence consisting of the base sequence (SEQ ID NO: 1) of NC_045512.2, or a mutant thereof. The SARS-CoV-1 encompasses a virus having a genomic RNA sequence consisting of the base sequence of NC_004718.3, or a mutant thereof. The MERS-CoV encompasses a virus having a genomic RNA sequence consisting of the base sequence of NC_019843.3, or a mutant thereof.

Herein, the mutant refers to a progeny having a new property formed by a mutation in which genetic information is partially changed due to partial misreading or recombination in the course of replication of a viral gene. Although the property of the mutant is partially changed under the influence of the changed genetic information, the original species of the virus is not changed. As for the family *Coronaviridae,* viruses that share 90% or more of the amino acid sequence of conserved replicase region reportedly belong to the same viral species (cf., the section Coronaviridae of Nidovirales of ICTV 9th report (2011) (https://talk.ictvonline.org/ictv-reports/ictv_9th_report/positive-sense-rna-viruses-2011/w/posrna_viruses/222/coronaviridae)) .

In one embodiment, the antisense oligonucleotide of the present invention is capable of exerting an effect on not only known SARS-related coronavirus (SARSr-CoV) but unknown SARS-related coronavirus. As used herein, the SARS-related coronavirus is a coronavirus that infects mammals including humans and bats and means an enveloped virus with a positive-sense single-stranded RNA comprised in the genus *Betacoronavirus* (group 2 coronavirus). The SARS-related coronavirus invades cells by exploiting angiotensin-converting enzyme 2 (ACE2) receptor.

In one embodiment, the antiviral effect means an effect of suppressing the growth of a virus and/or reducing the infectivity of the virus. Whether or not the antisense oligonucleotide of the present invention has the antiviral effect can be tested as described in Examples herein. For example, the presence or absence of the antiviral effect can be measured by preparing a plasmid for expression of a nucleic acid comprising a target sequence, introducing the plasmid and the antisense oligonucleotide of the present invention into a cell, and examining whether or not the amount of the nucleic acid comprising the target sequence expressed from the cell is decreased (*e.g*., by 5% or more, 10% or more, or 20% or more) as compared with the case of introducing a negative control nucleic acid. Alternatively, the presence or absence of the antiviral effect can be measured by introducing a virus and the antisense oligonucleotide of the present invention into a cell, culturing the cell, and then examining whether or not the amount of the virus or a nucleic acid derived therefrom in the cell or in a cell culture supernatant is decreased (*e.g*., by 5% or more, 10% or more, or 20% or more) as compared with the case of introducing a negative control nucleic acid.

In one embodiment, the antisense oligonucleotide of the present invention has at least 10%, at least 20%, at least 30%, at least 40%, at least 50%, at least 60%, at least 70%, at least 80%, or at least 90% knockdown activity at either 1 µM or 3 µM or on either the 5' or 3' side of Flue correction in a test on the knockdown activity of a gapmer using a SARS-CoV-2 expression plasmid when tested as described in Example 1, or has the same target sequence as that of an antisense oligonucleotide having this knockdown activity.

In one embodiment, the antisense oligonucleotide of the present invention has at least 5%, at least 10%, at least 20%, at least 30%, at least 40%, at least 50%, at least 60%, at least 70%, at least 80%, or at least 90% knockdown activity at any concentration of 2, 3, 6, 8, or 10 µM in the knockdown activity measurement of a gapmer using SARS-CoV-2 virus when tested as described in Example 2, or has the same target sequence as that of an antisense oligonucleotide having this knockdown activity.

The antisense oligonucleotide of the present invention targets a sequence in at least one target region selected from the group consisting of 5' UTR region, nsp1 region, nsp3 region, nsp4 region, 3C-like proteinase region, nsp6 region, nsp7 region, nsp8 region, nsp9 region, nsp10 region, RNA-dependent RNA polymerase region, helicase region, 3'-to-5' exonuclease region, endoRNase region, 2'-O-ribose methyltransferase region, S region comprising S1 region and S2 region, ORF3a region, E region, M region, ORF7b region, N region, ORF10 region, and 3' UTR region in genomic RNA of SARS-CoV-2.

The sequence of the genomic RNA of SARS-CoV-2 and the sequence of each region in the genomic RNA can be easily determined with reference to a database (*e.g.*, NCBI). The sequence of the genomic RNA of SARS-CoV-2 can be, for example, the base sequence (SEQ ID NO: 1) of NC_045512.2. In the base sequence of SEQ ID NO: 1, the 5' UTR region can be a base sequence from positions 1 to 265 of SEQ ID NO: 1; the nsp1 region can be a base sequence from positions 266 to 805 of SEQ ID NO: 1; the nsp3 region can be a base sequence from positions 2720 to 8554 of SEQ ID NO: 1; the nsp4 region can be a base sequence from positions 8555 to 10054 of SEQ ID NO: 1; the 3C-like proteinase region can be a base sequence from positions 10055 to 10972 of SEQ ID NO: 1; the nsp6 region can be a base sequence from positions 10973 to 11842 of SEQ ID NO: 1; the nsp7 region can be a base sequence from positions 11843 to 12091 of SEQ ID NO: 1; the nsp8 region can be a base sequence from positions 12092 to 12685 of SEQ ID NO: 1; the nsp9 region can be a base sequence from positions 12686 to 13024 of SEQ ID NO: 1; the nsp10 region can be a base sequence from positions 13025 to 13441 of SEQ ID NO: 1; the RNA-dependent RNA polymerase region can be a base sequence from positions 13442 to 16236 of SEQ ID NO: 1; the helicase region can be a base sequence from positions 16237 to 18039 of SEQ ID NO: 1; the 3'-to-5' exonuclease region can be a base sequence from positions 18040 to 19620 of SEQ ID NO: 1; the endoRNase region can be a base sequence from positions 19621 to 20658 of SEQ ID NO: 1; the 2'-O-ribose methyltransferase region can be a base sequence from positions 20659 to 21552 of SEQ ID NO: 1; the S region can be a base sequence from positions 21563 to 25384 of SEQ ID NO: 1 (the S1 region can be a base sequence from positions 21599 to 23617 of SEQ ID NO: 1, and the S2 region can be a base sequence from positions 23618 to 25381 of SEQ ID NO: 1); the ORF3a region can be a base sequence from positions 25393 to 26220 of SEQ ID NO: 1; the E region can be a base sequence from positions 26245 to 26472 of SEQ ID NO: 1; the M region can be a base sequence from positions 26523 to 27191 of SEQ ID NO: 1; the ORF7b region can be a base sequence from positions 27756 to 27887 of SEQ ID NO: 1; the N region can be a base sequence from positions 28274 to 29533 of SEQ ID NO: 1; the ORF10 region can be a base sequence from positions 29558 to 29674 of SEQ ID NO: 1; and the 3' UTR region can be a base sequence from positions 29675 to 29903 of SEQ ID NO: 1.

In one embodiment, the antisense oligonucleotide of the present invention targets a conserved region in the sequences of genomic RNA of SARS-CoV-2 and genomic RNA of SARS-CoV-1 and is therefore capable of exerting an antiviral effect on the genus *Betacoronavirus* comprising SARS-CoV-2, SARS-CoV-1 and MERS-CoV. In one embodiment, the antisense oligonucleotide of the present invention is complementary to a nucleic acid comprising at least 10 consecutive bases in a conserved region in the sequences of genomic RNA of SARS-CoV-2 and genomic RNA of SARS-CoV-1 among the target regions described above, for example, at least one target region selected from the group consisting of positions 43 to 89, positions 242 to 279, positions 290 to 312, positions 455 to 477, positions 704 to 723, positions 3352 to 3372, positions 5384 to 5403, positions 6071 to 6090, positions 6406 to 6427, positions 6797 to 6815, positions 7532 to 7551, positions 8707 to 8724, positions 10292 to 10309, positions 10406 to 10431, positions 10484 to 10506, positions 11609 to 11630, positions 12023 to 12045, positions 12170 to 12188, positions 12212 to 12234, positions 12314 to 12339, positions 12401 to 12420, positions 12589 to 12608, positions 12839 to 12867, positions 12898 to 12922, positions 12965 to 12990, positions 13151 to 13175, positions 13271 to 13290, positions 13363 to 13386, positions 13458 to 13502, positions 13642 to 13661, positions 13672 to 13691, positions 13762 to 13790, positions 13894 to 13916, positions 14050 to 14069, positions 14290 to 14312, positions 14512 to 14531, positions 14654 to 14687, positions 14698 to 14717, positions 14750 to 14777, positions 14824 to 14846, positions 14854 to 14873, positions 14878 to 14909, positions 14953 to 14990, positions 14992 to 15026, positions 15037 to 15061, positions 15063 to 15140, positions 15172 to 15198, positions 15278 to 15299, positions 15454 to 15479, positions 15496 to 15518, positions 15520 to 15539, positions 15622 to 15644, positions 15791 to 15809, positions 15829 to 15859, positions 15886 to 15905, positions 15929 to 15950, positions 15985 to 16011, positions 16051 to 16085, positions 16189 to 16220, positions 16430 to 16451, positions 16636 to 16655, positions 16822 to 16845, positions 17015 to 17051, positions 17080 to 17103, positions 17137 to 17156, positions 17215 to 17234, positions 17254 to 17277, positions 17564 to 17600, positions 17748 to 17765, positions 17773 to 17792, positions 17830 to 17849, positions 17859 to 17876, positions 18094 to 18111, positions 18196 to 18218, positions 18253 to 18278, positions 18370 to 18387, positions 19568 to 19595, positions 19622 to 19639, positions 19780 to 19802, positions 20107 to 20130, positions 20776 to 20795, positions 20797 to 20816, positions 20888 to 20909, positions 21453 to 21472, positions 21502 to 21524, positions 22550 to 22569, positions 22814 to 22836, positions 23093 to 23113, positions 23956 to 23976, positions 24302 to 24324, positions 24446 to 24465, positions 24467 to 24489, positions 24620 to 24651, positions 24662 to 24684, positions 24962 to 24982, positions 25104 to 25128, positions 25364 to 25384, positions 25502 to 25520, positions 26287 to 26325, positions 26574 to 26604, positions 26782 to 26800, positions 27093 to 27111, positions 27771 to 27794, positions 27806 to 27823, positions 28270 to 28294, positions 28397 to 28418, positions 28509 to 28538, positions 28744 to 28784, positions 28799 to 28820, positions 28946 to 28972, positions 28986 to 29005, positions 29010 to 29031, positions 29102 to 29130, positions 29174 to 29196, positions 29354 to 29373, positions 29615 to 29634, positions 29712 to 29731, and positions 29787 to 29867 of SEQ ID NO: 1.

In one embodiment, the antisense oligonucleotide of the present invention is complementary to a nucleic acid comprising at least 10 consecutive bases in at least one target region selected from the group consisting of positions 47 to 66, positions 242 to 261, positions 259 to 278, positions 292 to 311, positions 456 to 475, positions 704 to 723, positions 3353 to 3372, positions 5384 to 5403, positions 6071 to 6090, positions 6406 to 6425, positions 6407 to 6426, positions 6408 to 6427, positions 6797 to 6815, positions 7532 to 7551, positions 8707 to 8724, positions 10292 to 10309, positions 10407 to 10426, positions 10409 to 10428, positions 10487 to 10506, positions 11609 to 11628, positions 12025 to 12044, positions 12170 to 12188, positions 12214 to 12233, positions 12316 to 12335, positions 12401 to 12420, positions 12589 to 12608, positions 12843 to 12862, positions 12900 to 12919, positions 12969 to 12988, positions 13153 to 13172, positions 13271 to 13290, positions 13366 to 13385, positions 13462 to 13481, positions 13481 to 13500, positions 13642 to 13661, positions 13672 to 13691, positions 13767 to 13786, positions 13894 to 13913, positions 14050 to 14069, positions 14292 to 14311, positions 14512 to 14531, positions 14656 to 14675, positions 14668 to 14687, positions 14698 to 14717, positions 14753 to 14772, positions 14826 to 14845, positions 14854 to 14873, positions 14879 to 14898, positions 14889 to 14908, positions 14962 to 14981, positions 14971 to 14990, positions 14992 to 15011, positions 15007 to 15026, positions 15040 to 15059, positions 15064 to 15083, positions 15091 to 15107, positions 15092 to 15111, positions 15120 to 15139, positions 15172 to 15191, positions 15173 to 15192, positions 15174 to 15193, positions 15175 to 15194, positions 15179 to 15198, positions 15280 to 15299, positions 15280 to 15296, positions 15457 to 15476, positions 15496 to 15515, positions 15497 to 15516, positions 15498 to 15517, positions 15499 to 15518, positions 15520 to 15539, positions 15623 to 15642, positions 15791 to 15809, positions 15834 to 15853, positions 15886 to 15905, positions 15931 to 15950, positions 15988 to 16007, positions 16052 to 16071, positions 16066 to 16085, positions 16189 to 16208, positions 16192 to 16211, positions 16193 to 16212, positions 16194 to 16213, positions 16195 to 16214, positions 16196 to 16215, positions 16198 to 16217, positions 16201 to 16220, positions 16432 to 16451, positions 16636 to 16655, positions 16825 to 16844, positions 17016 to 17035, positions 17032 to 17051, positions 17080 to 17099, positions 17083 to 17102, positions 17084 to 17103, positions 17137 to 17156, positions 17215 to 17234, positions 17257 to 17276, positions 17564 to 17583, positions 17565 to 17584, positions 17566 to 17585, positions 17567 to 17586, positions 17569 to 17588, positions 17573 to 17592, positions 17576 to 17595, positions 17580 to 17599, positions 17748 to 17765, positions 17773 to 17792, positions 17830 to 17849, positions 17859 to 17876, positions 18094 to 18111, positions 18197 to 18216, positions 18257 to 18276, positions 18370 to 18387, positions 19572 to 19591, positions 19622 to 19639, positions 19780 to 19799, positions 19783 to 19802, positions 20107 to 20126, positions 20108 to 20127, positions 20109 to 20128, positions 20110 to 20129, positions 20111 to 20130, positions 20776 to 20795, positions 20797 to 20816, positions 20889 to 20908, positions 21453 to 21472, positions 21502 to 21521, positions 21503 to 21522, positions 21504 to 21523, positions 22550 to 22569, positions 22817 to 22836, positions 23093 to 23112, positions 23957 to 23976, positions 24303 to 24322, positions 24446 to 24465, positions 24469 to 24488, positions 24620 to 24639, positions 24632 to 24651, positions 24665 to 24684, positions 24962 to 24981, positions 25107 to 25126, positions 25365 to 25384, positions 25502 to 25520, positions 26287 to 26306, positions 26305 to 26324, positions 26579 to 26598, positions 26782 to 26800, positions 27093 to 27111, positions 27774 to 27793, positions 27806 to 27823, positions 28274 to 28293, positions 28398 to 28417, positions 28514 to 28533, positions 28744 to 28763, positions 28745 to 28764, positions 28746 to 28765, positions 28747 to 28766, positions 28748 to 28767, positions 28752 to 28771, positions 28756 to 28775, positions 28757 to 28776, positions 28758 to 28777, positions 28759 to 28778, positions 28762 to 28781, positions 28765 to 28784, positions 28799 to 28818, positions 28950 to 28969, positions 28986 to 29005, positions 29010 to 29029, positions 29106 to 29125, positions 29176 to 29195, positions 29354 to 29373, positions 29615 to 29634, positions 29712 to 29731, and positions 29789 to 29808 of SEQ ID NO: 1.

In one embodiment, the antisense oligonucleotide of the present invention comprises
(a) a base sequence selected from the group consisting of SEQ ID NOs: 2 to 177,
(b) a base sequence having addition, deletion, or substitution of one or several base(s) in the base sequence selected from the group consisting of SEQ ID NOs: 2 to 177, or
(c) a base sequence having at least 80%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% sequence identity to the base sequence selected from the group consisting of SEQ ID NOs: 2 to 177, or
consists of any of these sequences. In one embodiment, the antisense oligonucleotide of the present invention comprises any base sequence (a) or consists of the sequence.

As used herein, the term "several" for the base sequence having addition, deletion, or substitution of one or several base(s) means 2, 3, 4, 5, 6, 7, 8, 9, or 10.

In one embodiment, the at least one target region of the antisense oligonucleotide of the present invention is selected from the group consisting of, nsp1 region, nsp3 region, 3C-like proteinase region, nsp8 region, RNA-dependent RNA polymerase region, helicase region, 3'-to-5' exonuclease region, endoRNase region, 2'-O-ribose methyltransferase region, S region comprising S1 region and S2 region, M region, ORF7b region, N region, ORF10 region, and 3' UTR region in genomic RNA of SARS-CoV-2. For example, the antisense oligonucleotide of the present invention is complementary to a nucleic acid comprising at least 10 consecutive bases in at least one target region selected from the group consisting of positions 704 to 723, positions 5384 to 5403, positions 6407 to 6426, positions 10409 to 10428, positions 10487 to 10506, positions 12170 to 12188, positions 12214 to 12233, positions 12316 to 12335, positions 12401 to 12420, positions 13642 to 13661, positions 13672 to 13691, positions 13767 to 13786, positions 13894 to 13913, positions 14050 to 14069, positions 14512 to 14531, positions 14656 to 14675, positions 14668 to 14687, positions 14753 to 14772, positions 14826 to 14845, positions 14879 to 14898, positions 14889 to 14908, positions 14962 to 14981, positions 14992 to 15011, positions 15007 to 15026, positions 15040 to 15059, positions 15064 to 15083, positions 15091 to 15107, positions 15092 to 15111, positions 15120 to 15139, positions 15173 to 15192, positions 15175 to 15194, positions 15179 to 15198, positions 15280 to 15299, positions 15280 to 15296, positions 15457 to 15476, positions 15496 to 15515, positions 15498 to 15517, positions 15791 to 15809, positions 15834 to 15853, positions 15886 to 15905, positions 15931 to 15950, positions 15988 to 16007, positions 16052 to 16071, positions 16066 to 16085, positions 16189 to 16208, positions 16192 to 16211, positions 16195 to 16214, positions 16201 to 16220, positions 16432 to 16451, positions 16636 to 16655, positions 17016 to 17035, positions 17032 to 17051, positions 17083 to 17102, positions 17084 to 17103, positions 17257 to 17276, positions 17565 to 17584, positions 17576 to 17595, positions 17580 to 17599, positions 17748 to 17765, positions 17773 to 17792, positions 17830 to 17849, positions 18094 to 18111, positions 18197 to 18216, positions 18257 to 18276, positions 19622 to 19639, positions 19783 to 19802, positions 20108 to 20127, positions 20111 to 20130, positions 20776 to 20795, positions 20797 to 20816, positions 20889 to 20908, positions 21502 to 21521, positions 21504 to 21523, positions 24469 to 24488, positions 24620 to 24639, positions 24632 to 24651, positions 24665 to 24684, positions 24962 to 24981, positions 25107 to 25126, positions 26782 to 26800, positions 27806 to 27823, positions 28274 to 28293, positions 28514 to 28533, positions 28745 to 28764, positions 28746 to 28765, positions 28756 to 28775, positions 28757 to 28776, positions 28758 to 28777, positions 28759 to 28778, positions 28765 to 28784, positions 28799 to 28818, positions 28950 to 28969, positions 29010 to 29029, positions 29106 to 29125, positions 29176 to 29195, positions 29354 to 29373, positions 29615 to 29634, and positions 29712 to 29731 of SEQ ID NO: 1.

In one embodiment, the antisense oligonucleotide of the present invention comprises
(a) a base sequence selected from the group consisting of SEQ ID NOs: 7, 9, 12, 19, 20, 23 to 26, 36 to 40, 42 to 44, 46, 47, 49 to 51, 53 to 59, 61, 63 to 68, 70, 74 to 82, 85, 88 to 90, 92, 93, 95, 96, 99, 101, 106 to 110, 112 to 114, 117, 119, 121, 124, 125 to 127, 129, 131, 138 to 143, 149, 152, 153, 155, 157, 158, 162 to 165, 167 to 169, and 171 to 176,
(b) a base sequence having addition, deletion, or substitution of one or several base(s) in the base sequence selected from the group consisting of SEQ ID NOs: 7, 9, 12, 19, 20, 23 to 26, 36 to 40, 42 to 44, 46, 47, 49 to 51, 53 to 59, 61, 63 to 68, 70, 74 to 82, 85, 88 to 90, 92, 93, 95, 96, 99, 101, 106 to 110, 112 to 114, 117, 119, 121, 124, 125 to 127, 129, 131, 138 to 143, 149, 152, 153, 155, 157, 158, 162 to 165, 167 to 169, and 171 to 176, or
(c) a base sequence having at least 80%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% sequence identity to the base sequence selected from the group consisting of SEQ ID NO: 7, 9, 12, 19, 20, 23 to 26, 36 to 40, 42 to 44, 46, 47, 49 to 51, 53 to 59, 61, 63 to 68, 70, 74 to 82, 85, 88 to 90, 92, 93, 95, 96, 99, 101, 106 to 110, 112 to 114, 117, 119, 121, 124, 125 to 127, 129, 131, 138 to 143, 149, 152, 153, 155, 157, 158, 162 to 165, 167 to 169, and 171 to 176, or
consists of any of these sequences. In one embodiment, the antisense oligonucleotide of the present invention comprises any base sequence (a) or consists of the sequence.

In one embodiment, the at least one target region of the antisense oligonucleotide of the present invention is selected from the group consisting of nsp3 region, RNA-dependent RNA polymerase region, helicase region, 3'-to-5' exonuclease region, endoRNase region, 2'-O-ribose methyltransferase region, S region comprising S1 region and S2 region, and N region in genomic RNA of SARS-CoV-2. For example, the antisense oligonucleotide of the present invention is complementary to a nucleic acid comprising at least 10 consecutive bases in at least one target region selected from the group consisting of positions 5384 to 5403, positions 6407 to 6426, positions 13642 to 13661, positions 13672 to 13691, positions 15173 to 15192, positions 15175 to 15194, positions 15179 to 15198, positions 15496 to 15515, positions 15498 to 15517, positions 16189 to 16208, positions 16192 to 16211, positions 16195 to 16214, positions 16201 to 16220, positions 17083 to 17102, positions 17084 to 17103, positions 17565 to 17584, positions 17576 to 17595, positions 18257 to 18276, positions 19783 to 19802, positions 20108 to 20127, positions 20111 to 20130, positions 21502 to 21521, positions 21504 to 21523, positions 24665 to 24684, positions 28745 to 28764, positions 28746 to 28765, positions 28756 to 28775, positions 28757 to 28776, positions 28758 to 28777, and positions 28759 to 28778 of SEQ ID NO: 1. In one embodiment, the antisense oligonucleotide of the present invention is complementary to a nucleic acid comprising at least 10 consecutive bases in at least one target region selected from the group consisting of positions 6407 to 6426, positions 15173 to 15192, positions 15496 to 15515, positions 15498 to 15517, positions 16195 to 16214, positions 17084 to 17103, positions 20111 to 20130, positions 21502 to 21521, positions 28757 to 28776, and positions 28758 to 28777 of SEQ ID NO: 1.

In one embodiment, the antisense oligonucleotide of the present invention comprises
(a) a base sequence selected from the group consisting of SEQ ID NOs: 9, 12, 36, 37, 61, 63, 64, 68, 70, 81, 82, 85, 88, 95, 96, 101, 106, 114, 119, 121, 124, 129, 131, 141, 157, 158, 162, 163, 164, and 165,
(b) a base sequence having addition, deletion, or substitution of one or several base(s) in the base sequence selected from the group consisting of SEQ ID NOs: 9, 12, 36, 37, 61, 63, 64, 68, 70, 81, 82, 85, 88, 95, 96, 101, 106, 114, 119, 121, 124, 129, 131, 141, 157, 158, 162, 163, 164, and 165, or
(c) a base sequence having at least 80% sequence identity to the base sequence selected from the group consisting of SEQ ID NOs: 9, 12, 36, 37, 61, 63, 64, 68, 70, 81, 82, 85, 88, 95, 96, 101, 106, 114, 119, 121, 124, 129, 131, 141, 157, 158, 162, 163, 164, and 165, or consists of any of these sequences. In one embodiment, the antisense oligonucleotide of the present invention comprises any base sequence (a) or consists of the sequence.

In one embodiment, the antisense oligonucleotide of the present invention comprises
(a) a base sequence selected from the group consisting of SEQ ID NOs: 12, 61, 68, 70, 85, 96, 124, 129, 163, and 164,
(b) a base sequence having addition, deletion, or substitution of one or several base(s) in the base sequence selected from the group consisting of SEQ ID NOs: 12, 61, 68, 70, 85, 96, 124, 129, 163, and 164, or
(c) a base sequence having at least 80%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to the base sequence selected from the group consisting of SEQ ID NOs: 12, 61, 68, 70, 85, 96, 124, 129, 163, and 164, or
consists of any of these sequences. In one embodiment, the antisense oligonucleotide of the present invention comprises any base sequence (a) or consists of the sequence.

In one embodiment, the antisense oligonucleotide of the present invention inhibits a function of the target region. As used herein, the phrase "inhibit a function of the target region" comprises one or more of RNaseH cleavage of RNA comprising the target region that has formed double strands with the antisense oligonucleotide through binding to the target region, inhibition of the replication of RNA comprising the target region, inhibition of the translation of the target region when the target region is translated, and inhibition of the transcription of RNA comprising the target region. As used herein, the target region may be present in subgenomic RNA in addition to genomic RNA. The term "subgenomic RNA" means RNA that is shorter than genomic RNA, is synthesized by RNA-dependent RNA polymerase using as a template a portion of (-)-strand RNA synthesized by RNA-dependent RNA polymerase with (+)-strand genomic RNA as a template, and works as mRNA for viral protein synthesis (translation).

The antisense oligonucleotide of the present invention can generally be produced in accordance with a method described in, for example, W. Brad Wan et al., Nucleic Acid Research, Vol. 42, No. 22, 13456 (2014). Also, the antisense oligonucleotide of the present invention may be easily synthesized using various automated synthesizers (e.g., AKTA oligopilot plus 10/100 (GE Healthcare)). Alternatively, the synthesis may also be entrusted to a third-party organization (e.g., Promega Inc. or Takara Co.), etc.

In one embodiment, the present invention relates to a pharmaceutical composition comprising at least one antisense oligonucleotide of the present invention or pharmaceutically acceptable salt or hydrate thereof. In administration of the antisense oligonucleotide of the present invention to a subject, the pharmaceutical composition of the present invention may comprise a carrier to promote delivery of the antisense oligonucleotide. Such a carrier is not particularly limited as far as it is pharmaceutically acceptable, and examples include cationic carriers such as cationic liposomes, cationic polymers, etc., or carriers using viral envelope. Examples of the cationic liposomes include, for example, liposomes composed of 2-O-(2-diethylaminoethyl)carbamoyl-1,3-O-dioleoylglycerol and phospholipids as the essential constituents (hereinafter referred to as "liposome A"), Oligofectamine(R) (manufactured by Invitrogen Corp.), Lipofectin(R) (manufactured by Invitrogen Corp.), Lipofectamine(R) (manufactured by Invitrogen Corp.), Lipofectamine 2000(R) (manufactured by Invitrogen Corp.), DMRIE-C(R) (manufactured by Invitrogen Corp.), GeneSilencer(R) (manufactured by Gene Therapy Systems, Inc.), TransMessenger(R) (manufactured by Qiagen), TransIT TKO(R) (manufactured by Mirus), and Nucleofector II (manufactured by Lonza K.K.). Examples of cationic polymers include JetSI(R) (manufactured by Qbiogene, Inc.) and Jet-PEI(R) (polyethylenimine, manufactured by Qbiogene, Inc.). An example of carriers using viral envelop includes GenomeOne(R) (HVJ-E liposome, manufactured by Ishihara Sangyo Kaisha, Ltd.). Alternatively, the medical devices described in Japanese Patent No. 2924179 and the cationic carriers described in Japanese Domestic Re-Publication PCT Nos. 2006/129594 and 2008/096690 may be used as well.

In one embodiment, the antisense oligonucleotide of the present invention may be conjugated with a lipid or the like in the pharmaceutical composition in order to promote delivery of the antisense oligonucleotide. The antisense oligonucleotide of the present invention can be conjugated with cholesterol, as described in, for example, Bijsterbosch, M.K. et al., (2000) Nucleic Acid Res., 28, 2717-2725.

The pharmaceutical composition of the present invention may comprise pharmaceutically acceptable additives in addition to the antisense oligonucleotide or pharmaceutically acceptable salt or hydrate thereof and optionally the carrier described above. Examples of such additives are emulsification aids (e.g., fatty acids having 6 to 22 carbon atoms and their pharmaceutically acceptable salts, albumin, and dextran), stabilizers (e.g., cholesterol, phosphatidic acid, mannitol, and sorbitol), isotonizing agents (e.g., sodium chloride, glucose, maltose, lactose, sucrose, and trehalose), and pH controlling agents (e.g., hydrochloric acid, sulfuric acid, phosphoric acid, acetic acid, sodium hydroxide, potassium hydroxide, and triethanolamine). One or more of these additives can be used. The content of the additive in the composition of the present invention is appropriately 90% by weight or less, preferably 70% by weight or less, more preferably 50% by weight or less.

A method for preparing the pharmaceutical composition of the present invention is not limited, and the pharmaceutical composition of the present invention can be prepared, for example, by adding the antisense oligonucleotide of the present invention to a carrier dispersion and adequately stirring the mixture. Additives may be added at an appropriate step either before or after addition of the antisense oligonucleotide of the present invention. An aqueous solvent that can be used in adding the antisense oligonucleotide of the present invention is not particularly limited as far as it is pharmaceutically acceptable, and examples of the aqueous solvent include injectable water or injectable distilled water, electrolyte fluid such as physiological saline, etc., and sugar fluid such as glucose fluid, maltose fluid, etc. Those skilled in the art can appropriately choose conditions for pH and temperature for such case.

The pharmaceutical composition of the present invention may be prepared into, for example, a liquid form and its lyophilized preparation. The lyophilized preparation can be prepared by lyophilizing the composition of the present invention in a liquid form in a conventional manner. The lyophilization can be performed, for example, by appropriately sterilizing the composition of the present invention in a liquid form, dispensing an aliquot into a vial container, performing preliminary freezing for 2 hours at conditions within the range of about -40°C to -20°C, performing a primary drying within the range of about 0°C to 10°C under reduced pressure, and then performing a secondary drying within the range of about 15°C to 25°C under reduced pressure. In general, the lyophilized preparation of the composition of the present invention can be then obtained by replacing the gas of the vial with nitrogen gas and capping.

The lyophilized preparation of the pharmaceutical composition of the present invention can be used in general upon reconstitution by adding an optional suitable solution (reconstitution liquid) and redissolving the preparation. Such a reconstitution liquid includes injectable water, physiological saline, and other infusion fluids. A volume of the reconstitution liquid may vary depending on the intended use, etc., is not particularly limited, and is suitably 0.5-fold to 2-fold greater than the volume prior to lyophilization or no more than 500 mL.

A dose of the composition according to the present invention to be administered may be controlled by taking the following factors into account: the type of the comprised antisense oligonucleotide or pharmaceutically acceptable salt or hydrate thereof according to the present invention; the dosage form of the composition; patients' conditions including age, body weight, etc.; administration route; and the characteristics and symptoms of the disease. A daily dose calculated as the amount of the antisense oligonucleotide of the present invention can be, for example, 0.01 mg to 200 mg or 0.1 mg to 20 mg per kg body weight, preferably 0.2 mg to 10 mg per kg body weight, more preferably 0.5 mg to 4 mg per kg body weight, still more preferably 1 mg to 2 mg per kg body weight, for administration. The number of times of administration and a frequency of administration are not limited, and the administration may be performed, for example, only once or may be performed a total of a plurality of times (*e.g*., a total of twice) including another or more doses after several days (*e.g.*, on the next day to within 1 week) of the initial dose. For a plurality of doses, the frequency of administration is once every day to every 3 days, once every 4 to 6 days, once a week, or once every 2 to 3 weeks. These numerical vales may vary depending on type of the target disease, administration route and target molecule. Therefore, a dose or a frequency of administration lower than the range may be sufficient in some occasion and conversely, a dose or a frequency of administration higher than the range may be required occasionally.

Administration route for the composition according to the present invention is not particularly limited so long as it is pharmaceutically acceptable route for administration, and can be chosen depending upon method of treatment. Examples thereof include intratracheal administration, transpulmonary administration, transnasal administration, intravenous administration, intraarterial administration, intramuscular administration, subcutaneous administration, oral administration, tissue administration, transdermal administration, etc. Also, dosage forms which are available for the composition of the present invention are not particularly limited, and include, for example, inhalations, various injections, oral agents, drips, ointments, lotions, etc.

The administration route for the pharmaceutical composition according to the present invention is preferably intratracheal administration, and the dosage forms which are available for the composition of the present invention are preferably inhalations, specifically, for example, liquid inhalations (*e.g*., administered using a nebulizer), powder inhalations (*e.g*., administered using DPI (dry powder inhaler)), or aerosols, and preferably liquid inhalations.

The subject to which the antisense oligonucleotide or the pharmaceutical composition of the present invention is administered includes, for example, mammals, for example, primates such as humans, laboratory animals such as rats, mice, and Norway rats, and livestock animals such as pigs, bovines, horses, and sheep, etc., and is preferably a human.

In one embodiment, the present invention relates to a method for treatment and/or prevention of infection with a virus selected from the group consisting of SARS-CoV-2, SARS-CoV-1, and MERS-CoV, which comprises administering to a subject an effective amount of the antisense oligonucleotide or the pharmaceutically acceptable salt or hydrate thereof or the pharmaceutical composition according to the present invention. The pharmaceutical composition and the dose and administration route, etc. of the pharmaceutical composition according to the present embodiment are as described herein.

As used herein, the treatment of viral infection encompasses one or more of alleviation, improvement, and remission of a disease caused by the virus or symptoms thereof (*e.g*., one or more of dyspnea, fever, dry cough, headache, chill, and muscle pain). As used herein, the prevention of viral infection encompasses reduction of a risk resulting from a disease caused by the virus or symptoms thereof.

### Examples

Hereinafter, the present invention will be described in more detail with reference to Examples and Test Examples below. However, the present invention is not limited by the scope shown in Examples.

### <Example 1: Knockdown activity measurement of gapmer using SARS-CoV-2 expression plasmid>

The sequences of genomic RNA of SARS-CoV-2 (reference sequence; NC_045512.2) (SEQ ID NO: 1) and genomic RNA of SARS-CoV-1 (reference sequence; NC_004718.3) were compared to identify regions conserved between these two species. Base sequences from positions 43 to 89, positions 242 to 279, positions 290 to 312, positions 455 to 477, positions 704 to 723, positions 3352 to 3372, positions 5384 to 5403, positions 6071 to 6090, positions 6406 to 6427, positions 6797 to 6815, positions 7532 to 7551, positions 8707 to 8724, positions 10292 to 10309, positions 10406 to 10431, positions 10484 to 10506, positions 11609 to 11630, positions 12023 to 12045, positions 12170 to 12188, positions 12212 to 12234, positions 12314 to 12339, positions 12401 to 12420, positions 12589 to 12608, positions 12839 to 12867, positions 12898 to 12922, positions 12965 to 12990, positions 13151 to 13175, positions 13271 to 13290, positions 13363 to 13386, positions 13458 to 13502, positions 13642 to 13661, positions 13672 to 13691, positions 13762 to 13790, positions 13894 to 13916, positions 14050 to 14069, positions 14290 to 14312, positions 14512 to 14531, positions 14654 to 14687, positions 14698 to 14717, positions 14750 to 14777, positions 14824 to 14846, positions 14854 to 14873, positions 14878 to 14909, positions 14953 to 14990, positions 14992 to 15026, positions 15037 to 15061, positions 15063 to 15140, positions 15172 to 15198, positions 15278 to 15299, positions 15454 to 15479, positions 15496 to 15518, positions 15520 to 15539, positions 15622 to 15644, positions 15791 to 15809, positions 15829 to 15859, positions 15886 to 15905, positions 15929 to 15950, positions 15985 to 16011, positions 16051 to 16085, positions 16189 to 16220, positions 16430 to 16451, positions 16636 to 16655, positions 16822 to 16845, positions 17015 to 17051, positions 17080 to 17103, positions 17137 to 17156, positions 17215 to 17234, positions 17254 to 17277, positions 17564 to 17600, positions 17748 to 17765, positions 17773 to 17792, positions 17830 to 17849, positions 17859 to 17876, positions 18094 to 18111, positions 18196 to 18218, positions 18253 to 18278, positions 18370 to 18387, positions 19568 to 19595, positions 19622 to 19639, positions 19780 to 19802, positions 20107 to 20130, positions 20776 to 20795, positions 20797 to 20816, positions 20888 to 20909, positions 21453 to 21472, positions 21502 to 21524, positions 22550 to 22569, positions 22814 to 22836, positions 23093 to 23113, positions 23956 to 23976, positions 24302 to 24324, positions 24446 to 24465, positions 24467 to 24489, positions 24620 to 24651, positions 24662 to 24684, positions 24962 to 24982, positions 25104 to 25128, positions 25364 to 25384, positions 25502 to 25520, positions 26287 to 26325, positions 26574 to 26604, positions 26782 to 26800, positions 27093 to 27111, positions 27771 to 27794, positions 27806 to 27823, positions 28270 to 28294, positions 28397 to 28418, positions 28509 to 28538, positions 28744 to 28784, positions 28799 to 28820, positions 28946 to 28972, positions 28986 to 29005, positions 29010 to 29031, positions 29102 to 29130, positions 29174 to 29196, positions 29354 to 29373, positions 29615 to 29634, positions 29712 to 29731, and positions 29787 to 29867 of SEQ ID NO: 1 were found as regions conserved between the two species. Test substance gapmer Nos. G1 to G176 (SEQ ID NO: 2 to 177) were prepared by targeting sequences comprised in the conserved regions. In the test substance gapmer Nos. G1 to G176, all nucleosides of wing regions comprise a 2'-OMe (2'-O-CH₃) group. In Examples, gapmers suffixed with "MOE" in gapmer No. are 2'-O-MOE gapmers (gapmers in which all nucleosides of wing moieties comprise 2'-O-MOE (2'-O-CH₂CH₂OCH₃)). In Table 1-1 in Examples, all bonds between nucleosides are phosphorothioate bonds. In Examples, all oligonucleotides are gapmers of 5-10-5 type as a rule except for the following oligonucleotides.
19 mer 4-10-5 type: G110 and G114
19 mer 5-10-4 type: G11, G19, G61, and G115
18 mer 4-10-4 type: G13, G14, G81, G84, G85, G88, and G90
18 mer 5-9-4 type: G117
17 mer 4-9-4 type: 133-2'OMe, 133-2'MOE, 134-2'OMe, and 134-2'MOE

In these gapmers, the term "a-b-c type" means that each gapmer is constituted by a wing region of nucleotide length a, a gap region of nucleotide length b, and a wing region of nucleotide length c from the 5' end toward the 3' end.

In 2'-O-MOE gapmer, 2'-O-MOE-5-methyl C was used as C for the wing regions, and 5-methyl dC was used as C for the gap region.

Table 1-1 below shows the target sequences and the sequences of the test substance gapmer Nos. G1 to G176, etc.

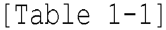

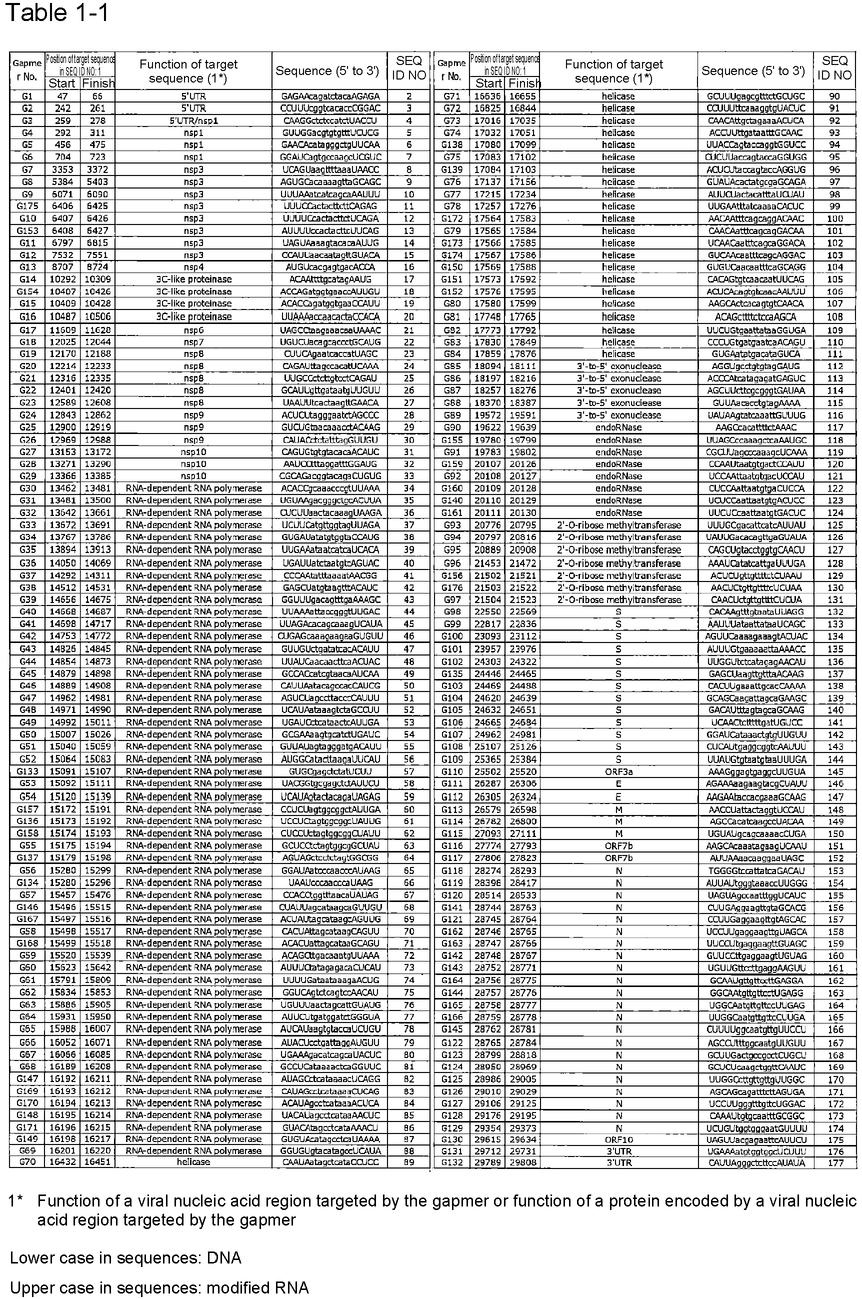

In Table 1-1, two described target sequences (5' UTR/nsp1 for G3) mean that the target sequence of the gapmer comprise two regions.

Table 1-2 below shows the target sequences and the sequences of PS/PS type test substance gapmer Nos. G58 PO/PS type-1, G58 PO/PS type-2, G144 PO/PS type-1, and G144 PO/PS type-2, etc. In the gapmers of 5-10-5 in Table 1-2, a bond between the second and third nucleosides and a bond between the fourth and fifth nucleosides from the 5' end of the 5' wing region are phosphodiester bonds, and a bond between the first and second nucleosides and a bond between the third and fourth nucleosides from the 5' end of the 3' wing region are phosphodiester bonds, while all bonds between the other nucleosides are phosphorothioate bonds (PO/PS type-2); or a bond between the second and third nucleosides, a bond between the third and fourth nucleosides, and a bond between the fourth and fifth nucleosides from the 5' end of the 5' wing region are phosphodiester bonds, and a bond between the first and second nucleosides, a bond between the second and third nucleosides, and a bond between the third and fourth nucleosides from the 5' end of the 3' wing region are phosphodiester bonds, while all bonds between the other nucleosides are phosphorothioate bonds (PO/PS type-1).

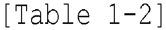

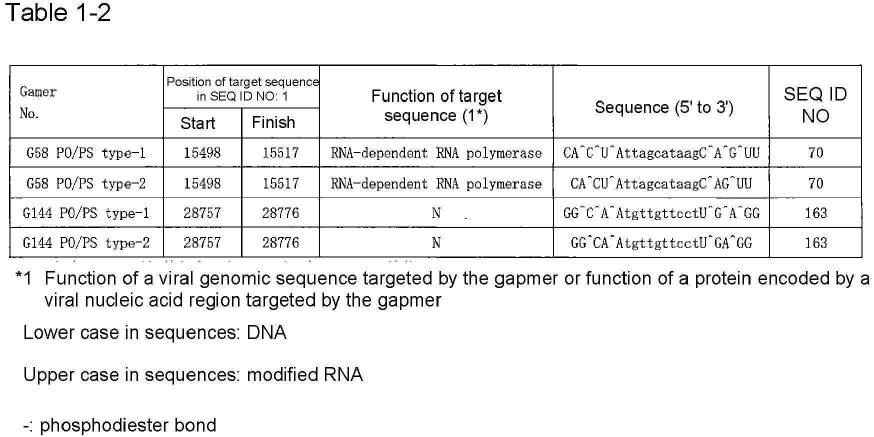

400 ng/sample (final concentration) of a SARS-CoV-2 expression plasmid, 4 ng/sample (final concentration) of a firefly luciferase (Flue) expression plasmid (control plasmid), and 1 or 3 µM (final concentration) test substance gapmer or negative control gapmer were introduced to 293T cells (obtained from ATCC) by electroporation using SF Cell Line 4D-Nucleofector X Kit (Lonza K.K.) and 4D-Nucleofector (Lonza K.K.) (16.4 µL of Nuleofector and 3.6 µL of supplement) according to DS-130 selected from a pulse program loaded in 4D-Nucleofector. The SARS-CoV-2 expression plasmid used was prepared by synthesizing as DNA artificial genes the genomic RNA of SARS-CoV-2 divided into ninth (Table 1-3), and subcloning these genes into expression plasmids pcDNA3.1. The artificial gene synthesis and the subcloning into expression plasmids were entrusted to Thermo Fisher Scientific Inc. However, since plasmid No. 4 was not available, SARS-CoV-2 (2019-nCoV) 3C-like proteinase/3CLpro Gene ORF cDNA clone expression plasmid (Sino Biological, Inc.) and SARS-CoV-2 (2019-nCoV) nsp8 Gene ORF cDNA clone expression plasmid (Sino Biological, Inc.) were used (described in "3CLpro" and "nsp8", respectively, in Table 1-3) in the activity evaluation of some gapmers. The control plasmid used was pGL4.50 [luc2/CMV/Hygro] Vector (Promega Inc.). The negative control gapmer used was the sequence of ASO #129700 (Wheeler et al., 2012. Nature 488: 111-5. doi: 10.1038/nature11362). The synthesis of the test substance gapmers and the negative control gapmer as gapmers comprising a 2'-OMe gapmer (gapmer in which all nucleosides of the wing moieties comprised 2'-OMe (2'-O-CH₃)) or a 2'-O-MOE gapmer (gapmer in which all nucleosides of the wing moieties comprised 2'-O-MOE (2'-O-CH₂CH₂OCH₃) was entrusted to Japan Bio Service Co.

2.0 × 10⁵ 293T cells were used per time of electroporation. The cells thus electroporated were inoculated to a 24-well plate. Two days after introduction of each gapmer and the corresponding plasmid, RNA was isolated from the cells using NucleoSpin RNA (Macherey-Nagel GmbH & Co. KG) and subjected to reverse-transcription (RT) reaction using High Capacity cDNA Reverse Transcription Kit with RNase Inhibitor (Thermo Fisher Scientific Inc.) and random primers attached to the kit. qPCR was performed with the RT reaction solution as a template using Fast SYBR Green Master Mix (Thermo Fisher Scientific Inc.) according to manufacturer's protocol to measure an expression level of SARS-CoV-2 RNA derived from the SARS-CoV-2 expression plasmid. Table 1-3 shows the primers used in the detection of SARS-CoV-2 RNA derived from the SARS-CoV-2 expression plasmid. The SARS-CoV-2 RNA expression level was corrected with an expression level of Flue RNA derived from the control plasmid or a PPIB RNA expression level of a housekeeping gene. Table 1-4 shows the primers used in the detection of Fluc RNA or PPIB RNA. The ratio of suppression of the SARS-CoV-2 RNA expression level resulting from the introduction of the test substance gapmer to the SARS-CoV-2 RNA expression level resulting from the introduction of the negative control gapmer (knockdown activity of the test substance gapmer) was analyzed. The results are shown in Table 1-5.

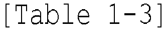

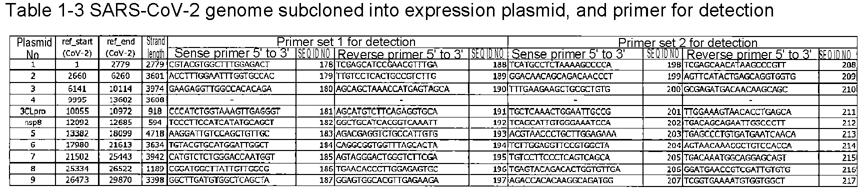

**[Table 1-4]**

| Table 1-4 Gene used in correction of SARS-CoV-2 expression level, and primer for detection | | | | |
|---|---|---|---|---|
| Gene for correction | Primer set for detection | | | |
| | Sense primer 5' to 3' | SEQ ID NO | Reverse primer 5' to 3' | SEQ ID NO |
| Flue | AACCAGCGCCATTCTGATCA | 218 | TCGGGGTTGTTAACGTAGCC | 220 |
| PPIB | CAGCAAATTCCATCGTG | 219 | CCGTAGTGCTTCAGTTT | 221 |

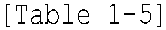

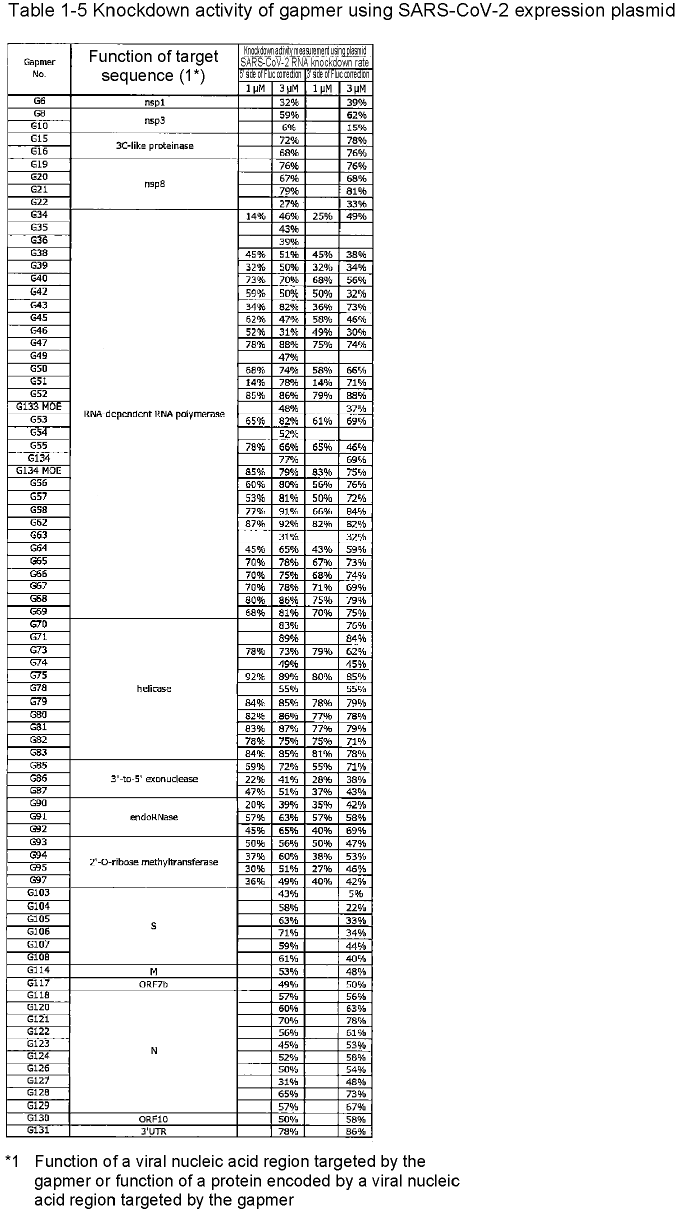

In the table, the terms "5' side of Flue correction" and "3' side of Flue correction" mean that which primer between 5' and 3' primers for detection (Table 1-3) is used to detect expression.

### <Example 2: Knockdown activity measurement of gapmer using SARS-CoV-2 virus>

2 to 10 µM (final concentration) test substance gapmer or negative control gapmer was introduced to stably human ACE2-expressing cells (293T-ACE2) (prepared by the infection of 293T cells with a human ACE2 expression lentivirus vector) by electroporation using Amxa Cell Line Nucleofector Kit V (Lonza K.K.) (73.6 µL of solution V and 16.4 µL/sample of supplement) and Nucleofector II (Amaxa) according to A-023 selected from a pulse program loaded in Nucleofector II. The negative control gapmer used was the sequence of ASO #129700 (Wheeler et al., 2012. Nature 488: 111-5. doi: 10.1038/nature11362). The test substance gapmers used were some unevaluated sequences among the gapmer Nos. G1 to G135 used in Example 1, and gapmer Nos. G136 to G176 additionally designed on the basis of activity data on gapmer Nos. 1 to 135. The synthesis of the test substance gapmers and the negative control gapmer as 2'-OMe gapmers or 2'-MOE gapmers was entrusted to Japan Bio Service Co. or Ajinomoto Bio-pharma Services.

6.0 × 10⁵ 293T-ACE2 cells were used per time of electroporation. The 293T-ACE2 thus electroporated were inoculated at 5.0 × 10⁴ cells/well to a 96-well plate. On the next day, a SARS-CoV-2 virus solution was added thereto at MOI = 0.01 so that the cells were infected with the virus. The SARS-CoV-2 virus used was recombinant SARS-CoV-2 virus (rSARS-CoV-2-ORF8-Nluc) in which nano-luciferase gene was incorporated in a coding region of an accessory protein ORF8 using the same approach as that described in the literature (Terada et al. 2019. J Virol 93: e01208-19. doi: 10.1128/JVI.01208-19) on the basis of a WK521 strain obtained from National Institute of Infectious Diseases, Japan. One hour after virus solution addition, the virus in the medium was removed by medium replacement. 48 hours after viral infection, the culture supernatant was recovered, and the cells were prepared into a cell lysate using Passive Lysis Buffer (Promega Inc.). Luciferase activity in the cell lysate was measured using Luciferase Assay System (Promega Inc.) according to the protocol attached thereto to evaluate the amount of the SARS-CoV-2 virus in the cells (measurement of the amount of intracellular virus). Also, the recovered culture supernatant was added at 5 µL/well to 293T-ACE2 cells separately inoculated at 3.0 × 10⁴ cells/well to a 96-well plate so that the cells were infected with the virus in the culture supernatant. Twenty-four hours after infection with the culture supernatant, the amount of the SARS-CoV-2 virus in the cells was evaluated in the same manner as in the first viral infection to evaluate the amount of infectious virus in the culture supernatant (measurement of infectious virus in the medium). Table 2 shows the knockdown activity of each test substance gapmer (the rate of suppression of the amount of infectious virus in the medium: the rate of suppression of the amount of infectious virus resulting from the introduction of the test substance gapmer to the amount of infectious virus resulting from the introduction of the negative control gapmer.

**[Table 2]**

| Table 2 Knockdown activity measurement of gapmer using SARS-CoV-2 virus | | | | | | |
|---|---|---|---|---|---|---|
| Gapmer No. | Function of target sequence (*1) | Rate of supp ression of amount of infectious virus in medium | | | | |
| | | 2 µM | 3 µM | 6 µM | 8 µM | 10 µM |
| G8 | nsp3 | 44% | | 67% | | |
| G10 | | | 57% | | 46% | |
| G32 | RNA-dependent RNA polymerase | | 14% | | | 49% |
| G33 | | | -8% | | | 45% |
| G136 | | | 52% | | | 93% |
| G136 MOE | | | 46% | | | 61% |
| G55 | | | 21% | | | 66% |
| G137 | | | 48% | | | 45% |
| G146 | | | 42% | | | 2% |
| G58 | | | 26% | | | 43% |
| G58 MOE | | | 64% | | | 64% |
| G68 | | | 31% | | | 68% |
| G147 | | | 51% | | | 76% |
| G148 | | | 65% | | | 79% |
| G69 | | 52% | | 51% | | |
| G75 | helicase | | 22% | | | 63% |
| G139 | | | 87% | | | 89% |
| G139 MOE | | | 66% | | | 54% |
| G79 | | | 38% | 66% | | |
| G152 | | | 29% | | | 42% |
| G87 | 3'-to-5' exonuclease | 22% | | 58% | | |
| G91 | endoRNase | | 9% | | | 47% |
| G92 | | | 8% | | | 73% |
| G161 MOE | | | 58% | | | 89% |
| G156 | 2'-O-ribose methyltransferase | | 44% | | | 39% |
| G97 | | | 9% | | | 45% |
| G106 | S | 14% | | 80% | | |
| G121 | N | | 11% | | | 69% |
| G162 | | | 17% | | | 43% |
| G164 | | | 22% | | | 46% |
| G144 | | | 65% | | | 67% |
| G144 MOE | | | 64% | | | 85% |
| G165 | | | 28% | | | 53% |
| G166 | | | 28% | | | 45% |
| *1 Function of a viral nucleic acid region targeted by the gapmer or function of a protein encoded by a viral nucleic acid region targeted by the gapmer | | | | | | |

## Claims

1. An antisense oligonucleotide consisting of 15 to 30 nucleotides or a pharmaceutically acceptable salt or hydrate thereof, the 15 to 30 nucleotides being complementary to a nucleic acid comprising at least 10 consecutive bases in at least one target region selected from the group consisting of 5' UTR region, nsp1 region, nsp3 region, nsp4 region, 3C-like proteinase region, nsp6 region, nsp7 region, nsp8 region, nsp9 region, nsp10 region, RNA-dependent RNA polymerase region, helicase region, 3'-to-5' exonuclease region, endoRNase region, 2'-O-ribose methyltransferase region, S region comprising S1 region and S2 region, ORF3a region, E region, M region, ORF7b region, N region, ORF10 region, and 3' UTR region in genomic RNA of SARS-CoV-2, wherein
the antisense oligonucleotide or the pharmaceutically acceptable salt or hydrate thereof has an antiviral effect on a virus selected from the group consisting of SARS-CoV-2, SARS-CoV-1, and MERS-CoV.

2. The antisense oligonucleotide or the pharmaceutically acceptable salt or hydrate thereof according to claim 1, wherein the antisense oligonucleotide is a gapmer comprising a center gap region and two wing regions flanking the gap region.

3. The antisense oligonucleotide or the pharmaceutically acceptable salt or hydrate thereof according to claim 1 or 2, wherein the virus is SARS-CoV-2 or SARS-CoV-1.

4. The antisense oligonucleotide or the pharmaceutically acceptable salt or hydrate thereof according to any one of claims 1 to 3, wherein the at least one target region is selected from the group consisting of nsp1 region, nsp3 region, 3C-like proteinase region, nsp8 region, RNA-dependent RNA polymerase region, helicase region, 3'-to-5' exonuclease region, endoRNase region, 2'-O-ribose methyltransferase region, S region comprising S1 region and S2 region, M region, ORF7b region, N region, ORF10 region, and 3' UTR region in genomic RNA of SARS-CoV-2.

5. The antisense oligonucleotide or the pharmaceutically acceptable salt or hydrate thereof according to any one of claims 1 to 4, wherein the at least one target region is selected from the group consisting of nsp3 region, RNA-dependent RNA polymerase region, helicase region, 3'-to-5' exonuclease region, endoRNase region, 2'-O-ribose methyltransferase region, S region comprising S1 region and S2 region, and N region in genomic RNA of SARS-CoV-2.

6. The antisense oligonucleotide or the pharmaceutically acceptable salt or hydrate thereof according to any one of claims 1 to 5, wherein the antisense oligonucleotide is complementary to a nucleic acid comprising at least 10 consecutive bases in at least one target region selected from the group consisting of positions 43 to 89, positions 242 to 279, positions 290 to 312, positions 455 to 477, positions 704 to 723, positions 3352 to 3372, positions 5384 to 5403, positions 6071 to 6090, positions 6406 to 6427, positions 6797 to 6815, positions 7532 to 7551, positions 8707 to 8724, positions 10292 to 10309, positions 10406 to 10431, positions 10484 to 10506, positions 11609 to 11630, positions 12023 to 12045, positions 12170 to 12188, positions 12212 to 12234, positions 12314 to 12339, positions 12401 to 12420, positions 12589 to 12608, positions 12839 to 12867, positions 12898 to 12922, positions 12965 to 12990, positions 13151 to 13175, positions 13271 to 13290, positions 13363 to 13386, positions 13458 to 13502, positions 13642 to 13661, positions 13672 to 13691, positions 13762 to 13790, positions 13894 to 13916, positions 14050 to 14069, positions 14290 to 14312, positions 14512 to 14531, positions 14654 to 14687, positions 14698 to 14717, positions 14750 to 14777, positions 14824 to 14846, positions 14854 to 14873, positions 14878 to 14909, positions 14953 to 14990, positions 14992 to 15026, positions 15037 to 15061, positions 15063 to 15140, positions 15172 to 15198, positions 15278 to 15299, positions 15454 to 15479, positions 15496 to 15518, positions 15520 to 15539, positions 15622 to 15644, positions 15791 to 15809, positions 15829 to 15859, positions 15886 to 15905, positions 15929 to 15950, positions 15985 to 16011, positions 16051 to 16085, positions 16189 to 16220, positions 16430 to 16451, positions 16636 to 16655, positions 16822 to 16845, positions 17015 to 17051, positions 17080 to 17103, positions 17137 to 17156, positions 17215 to 17234, positions 17254 to 17277, positions 17564 to 17600, positions 17748 to 17765, positions 17773 to 17792, positions 17830 to 17849, positions 17859 to 17876, positions 18094 to 18111, positions 18196 to 18218, positions 18253 to 18278, positions 18370 to 18387, positions 19568 to 19595, positions 19622 to 19639, positions 19780 to 19802, positions 20107 to 20130, positions 20776 to 20795, positions 20797 to 20816, positions 20888 to 20909, positions 21453 to 21472, positions 21502 to 21524, positions 22550 to 22569, positions 22814 to 22836, positions 23093 to 23113, positions 23956 to 23976, positions 24302 to 24324, positions 24446 to 24465, positions 24467 to 24489, positions 24620 to 24651, positions 24662 to 24684, positions 24962 to 24982, positions 25104 to 25128, positions 25364 to 25384, positions 25502 to 25520, positions 26287 to 26325, positions 26574 to 26604, positions 26782 to 26800, positions 27093 to 27111, positions 27771 to 27794, positions 27806 to 27823, positions 28270 to 28294, positions 28397 to 28418, positions 28509 to 28538, positions 28744 to 28784, positions 28799 to 28820, positions 28946 to 28972, positions 28986 to 29005, positions 29010 to 29031, positions 29102 to 29130, positions 29174 to 29196, positions 29354 to 29373, positions 29615 to 29634, positions 29712 to 29731, and positions 29787 to 29867 of SEQ ID NO: 1.

7. The antisense oligonucleotide or the pharmaceutically acceptable salt or hydrate thereof according to any one of claims 1 to 6, wherein the antisense oligonucleotide is complementary to a nucleic acid comprising at least 10 consecutive bases in at least one target region selected from the group consisting of positions 47 to 66, positions 242 to 261, positions 259 to 278, positions 292 to 311, positions 456 to 475, positions 704 to 723, positions 3353 to 3372, positions 5384 to 5403, positions 6071 to 6090, positions 6406 to 6425, positions 6407 to 6426, positions 6408 to 6427, positions 6797 to 6815, positions 7532 to 7551, positions 8707 to 8724, positions 10292 to 10309, positions 10407 to 10426, positions 10409 to 10428, positions 10487 to 10506, positions 11609 to 11628, positions 12025 to 12044, positions 12170 to 12188, positions 12214 to 12233, positions 12316 to 12335, positions 12401 to 12420, positions 12589 to 12608, positions 12843 to 12862, positions 12900 to 12919, positions 12969 to 12988, positions 13153 to 13172, positions 13271 to 13290, positions 13366 to 13385, positions 13462 to 13481, positions 13481 to 13500, positions 13642 to 13661, positions 13672 to 13691, positions 13767 to 13786, positions 13894 to 13913, positions 14050 to 14069, positions 14292 to 14311, positions 14512 to 14531, positions 14656 to 14675, positions 14668 to 14687, positions 14698 to 14717, positions 14753 to 14772, positions 14826 to 14845, positions 14854 to 14873, positions 14879 to 14898, positions 14889 to 14908, positions 14962 to 14981, positions 14971 to 14990, positions 14992 to 15011, positions 15007 to 15026, positions 15040 to 15059, positions 15064 to 15083, positions 15091 to 15107, positions 15092 to 15111, positions 15120 to 15139, positions 15172 to 15191, positions 15173 to 15192, positions 15174 to 15193, positions 15175 to 15194, positions 15179 to 15198, positions 15280 to 15299, positions 15280 to 15296, positions 15457 to 15476, positions 15496 to 15515, positions 15497 to 15516, positions 15498 to 15517, positions 15499 to 15518, positions 15520 to 15539, positions 15623 to 15642, positions 15791 to 15809, positions 15834 to 15853, positions 15886 to 15905, positions 15931 to 15950, positions 15988 to 16007, positions 16052 to 16071, positions 16066 to 16085, positions 16189 to 16208, positions 16192 to 16211, positions 16193 to 16212, positions 16194 to 16213, positions 16195 to 16214, positions 16196 to 16215, positions 16198 to 16217, positions 16201 to 16220, positions 16432 to 16451, positions 16636 to 16655, positions 16825 to 16844, positions 17016 to 17035, positions 17032 to 17051, positions 17080 to 17099, positions 17083 to 17102, positions 17084 to 17103, positions 17137 to 17156, positions 17215 to 17234, positions 17257 to 17276, positions 17564 to 17583, positions 17565 to 17584, positions 17566 to 17585, positions 17567 to 17586, positions 17569 to 17588, positions 17573 to 17592, positions 17576 to 17595, positions 17580 to 17599, positions 17748 to 17765, positions 17773 to 17792, positions 17830 to 17849, positions 17859 to 17876, positions 18094 to 18111, positions 18197 to 18216, positions 18257 to 18276, positions 18370 to 18387, positions 19572 to 19591, positions 19622 to 19639, positions 19780 to 19799, positions 19783 to 19802, positions 20107 to 20126, positions 20108 to 20127, positions 20109 to 20128, positions 20110 to 20129, positions 20111 to 20130, positions 20776 to 20795, positions 20797 to 20816, positions 20889 to 20908, positions 21453 to 21472, positions 21502 to 21521, positions 21503 to 21522, positions 21504 to 21523, positions 22550 to 22569, positions 22817 to 22836, positions 23093 to 23112, positions 23957 to 23976, positions 24303 to 24322, positions 24446 to 24465, positions 24469 to 24488, positions 24620 to 24639, positions 24632 to 24651, positions 24665 to 24684, positions 24962 to 24981, positions 25107 to 25126, positions 25365 to 25384, positions 25502 to 25520, positions 26287 to 26306, positions 26305 to 26324, positions 26579 to 26598, positions 26782 to 26800, positions 27093 to 27111, positions 27774 to 27793, positions 27806 to 27823, positions 28274 to 28293, positions 28398 to 28417, positions 28514 to 28533, positions 28744 to 28763, positions 28745 to 28764, positions 28746 to 28765, positions 28747 to 28766, positions 28748 to 28767, positions 28752 to 28771, positions 28756 to 28775, positions 28757 to 28776, positions 28758 to 28777, positions 28759 to 28778, positions 28762 to 28781, positions 28765 to 28784, positions 28799 to 28818, positions 28950 to 28969, positions 28986 to 29005, positions 29010 to 29029, positions 29106 to 29125, positions 29176 to 29195, positions 29354 to 29373, positions 29615 to 29634, positions 29712 to 29731, and positions 29789 to 29808 of SEQ ID NO: 1.

8. The antisense oligonucleotide or the pharmaceutically acceptable salt or hydrate thereof according to any one of claims 1 to 7, wherein the antisense oligonucleotide is complementary to a nucleic acid comprising at least 10 consecutive bases in at least one target region selected from the group consisting of positions 704 to 723, positions 5384 to 5403, positions 6407 to 6426, positions 10409 to 10428, positions 10487 to 10506, positions 12170 to 12188, positions 12214 to 12233, positions 12316 to 12335, positions 12401 to 12420, positions 13642 to 13661, positions 13672 to 13691, positions 13767 to 13786, positions 13894 to 13913, positions 14050 to 14069, positions 14512 to 14531, positions 14656 to 14675, positions 14668 to 14687, positions 14753 to 14772, positions 14826 to 14845, positions 14879 to 14898, positions 14889 to 14908, positions 14962 to 14981, positions 14992 to 15011, positions 15007 to 15026, positions 15040 to 15059, positions 15064 to 15083, positions 15091 to 15107, positions 15092 to 15111, positions 15120 to 15139, positions 15173 to 15192, positions 15175 to 15194, positions 15179 to 15198, positions 15280 to 15299, positions 15280 to 15296, positions 15457 to 15476, positions 15496 to 15515, positions 15498 to 15517, positions 15791 to 15809, positions 15834 to 15853, positions 15886 to 15905, positions 15931 to 15950, positions 15988 to 16007, positions 16052 to 16071, positions 16066 to 16085, positions 16189 to 16208, positions 16192 to 16211, positions 16195 to 16214, positions 16201 to 16220, positions 16432 to 16451, positions 16636 to 16655, positions 17016 to 17035, positions 17032 to 17051, positions 17083 to 17102, positions 17084 to 17103, positions 17257 to 17276, positions 17565 to 17584, positions 17576 to 17595, positions 17580 to 17599, positions 17748 to 17765, positions 17773 to 17792, positions 17830 to 17849, positions 18094 to 18111, positions 18197 to 18216, positions 18257 to 18276, positions 19622 to 19639, positions 19783 to 19802, positions 20108 to 20127, positions 20111 to 20130, positions 20776 to 20795, positions 20797 to 20816, positions 20889 to 20908, positions 21502 to 21521, positions 21504 to 21523, positions 24469 to 24488, positions 24620 to 24639, positions 24632 to 24651, positions 24665 to 24684, positions 24962 to 24981, positions 25107 to 25126, positions 26782 to 26800, positions 27806 to 27823, positions 28274 to 28293, positions 28514 to 28533, positions 28745 to 28764, positions 28746 to 28765, positions 28756 to 28775, positions 28757 to 28776, positions 28758 to 28777, positions 28759 to 28778, positions 28765 to 28784, positions 28799 to 28818, positions 28950 to 28969, positions 29010 to 29029, positions 29106 to 29125, positions 29176 to 29195, positions 29354 to 29373, positions 29615 to 29634, and positions 29712 to 29731 of SEQ ID NO: 1.

9. The antisense oligonucleotide or the pharmaceutically acceptable salt or hydrate thereof according to any one of claims 1 to 8, wherein the antisense oligonucleotide is complementary to a nucleic acid comprising at least 10 consecutive bases in at least one target region selected from the group consisting of positions 5384 to 5403, positions 6407 to 6426, positions 13642 to 13661, positions 13672 to 13691, positions 15173 to 15192, positions 15175 to 15194, positions 15179 to 15198, positions 15496 to 15515, positions 15498 to 15517, positions 16189 to 16208, positions 16192 to 16211, positions 16195 to 16214, positions 16201 to 16220, positions 17083 to 17102, positions 17084 to 17103, positions 17565 to 17584, positions 17576 to 17595, positions 18257 to 18276, positions 19783 to 19802, positions 20108 to 20127, positions 20111 to 20130, positions 21502 to 21521, positions 21504 to 21523, positions 24665 to 24684, positions 28745 to 28764, positions 28746 to 28765, positions 28756 to 28775, positions 28757 to 28776, positions 28758 to 28777, and positions 28759 to 28778 of SEQ ID NO: 1.

10. The antisense oligonucleotide or the pharmaceutically acceptable salt or hydrate thereof according to any one of claims 1 to 9, wherein the antisense oligonucleotide is complementary to a nucleic acid comprising at least 10 consecutive bases in at least one target region selected from the group consisting of positions 6407 to 6426, positions 15173 to 15192, positions 15496 to 15515, positions 15498 to 15517, positions 16195 to 16214, positions 17084 to 17103, positions 20111 to 20130, positions 21502 to 21521, positions 28757 to 28776, and positions 28758 to 28777 of SEQ ID NO: 1.

11. The antisense oligonucleotide or the pharmaceutically acceptable salt or hydrate thereof according to any one of claims 1 to 10, wherein the antisense oligonucleotide is complementary to a nucleic acid comprising at least 15 consecutive bases in the target region.

12. The antisense oligonucleotide or the pharmaceutically acceptable salt or hydrate thereof according to any one of claims 1 to 11, wherein the antisense oligonucleotide comprises
(a) a base sequence selected from the group consisting of SEQ ID NOs: 2 to 177,
(b) a base sequence having addition, deletion, or substitution of one or several base(s) in the base sequence selected from the group consisting of SEQ ID NOs: 2 to 177, or
(c) a base sequence having at least 80% sequence identity to the base sequence selected from the group consisting of SEQ ID NOs: 2 to 177, and
inhibits a function of the target region.

13. The antisense oligonucleotide or the pharmaceutically acceptable salt or hydrate thereof according to any one of claims 1 to 12, wherein the antisense oligonucleotide comprises
(a) a base sequence selected from the group consisting of SEQ ID NOs: 7, 9, 12, 19, 20, 23 to 26, 36 to 40, 42 to 44, 46, 47, 49 to 51, 53 to 59, 61, 63 to 68, 70, 74 to 82, 85, 88 to 90, 92, 93, 95, 96, 99, 101, 106 to 110, 112 to 114, 117, 119, 121, 124, 125 to 127, 129, 131, 138 to 143, 149, 152, 153, 155, 157, 158, 162 to 165, 167 to 169, and 171 to 176,
(b) a base sequence having addition, deletion, or substitution of one or several base(s) in the base sequence selected from the group consisting of SEQ ID NOs: 7, 9, 12, 19, 20, 23 to 26, 36 to 40, 42 to 44, 46, 47, 49 to 51, 53 to 59, 61, 63 to 68, 70, 74 to 82, 85, 88 to 90, 92, 93, 95, 96, 99, 101, 106 to 110, 112 to 114, 117, 119, 121, 124, 125 to 127, 129, 131, 138 to 143, 149, 152, 153, 155, 157, 158, 162 to 165, 167 to 169, and 171 to 176, or
(c) a base sequence having at least 80% sequence identity to the base sequence selected from the group consisting of SEQ ID NOs: 7, 9, 12, 19, 20, 23 to 26, 36 to 40, 42 to 44, 46, 47, 49 to 51, 53 to 59, 61, 63 to 68, 70, 74 to 82, 85, 88 to 90, 92, 93, 95, 96, 99, 101, 106 to 110, 112 to 114, 117, 119, 121, 124, 125 to 127, 129, 131, 138 to 143, 149, 152, 153, 155, 157, 158, 162 to 165, 167 to 169, and 171 to 176, and
inhibits a function of the target region.

14. The antisense oligonucleotide or the pharmaceutically acceptable salt or hydrate thereof according to any one of claims 1 to 13, wherein the antisense oligonucleotide comprises
(a) a base sequence selected from the group consisting of SEQ ID NOs: 9, 12, 36, 37, 61, 63, 64, 68, 70, 81, 82, 85, 88, 95, 96, 101, 106, 114, 119, 121, 124, 129, 131, 141, 157, 158, 162, 163, 164, and 165,
(b) a base sequence having addition, deletion, or substitution of one or several base(s) in the base sequence selected from the group consisting of SEQ ID NOs: 9, 12, 36, 37, 61, 63, 64, 68, 70, 81, 82, 85, 88, 95, 96, 101, 106, 114, 119, 121, 124, 129, 131, 141, 157, 158, 162, 163, 164, and 165, or
(c) a base sequence having at least 80% sequence identity to the base sequence selected from the group consisting of SEQ ID NOs: 9, 12, 36, 37, 61, 63, 64, 68, 70, 81, 82, 85, 88, 95, 96, 101, 106, 114, 119, 121, 124, 129, 131, 141, 157, 158, 162, 163, 164, and 165, and
inhibits a function of the target region.

15. The antisense oligonucleotide or the pharmaceutically acceptable salt or hydrate thereof according to any one of claims 1 to 14, wherein the antisense oligonucleotide comprises
(a) a base sequence selected from the group consisting of SEQ ID NOs: 12, 61, 68, 70, 85, 96, 124, 129, 163, and 164,
(b) a base sequence having addition, deletion, or substitution of one or several base(s) in the base sequence selected from the group consisting of SEQ ID NOs: 12, 61, 68, 70, 85, 96, 124, 129, 163, and 164, or
(c) a base sequence having at least 80% sequence identity to the base sequence selected from the group consisting of SEQ ID NOs: 12, 61, 68, 70, 85, 96, 124, 129, 163, and 164, and
inhibits a function of the target region.

16. The antisense oligonucleotide or the pharmaceutically acceptable salt or hydrate thereof according to any one of claims 11 to 15, wherein the antisense oligonucleotide comprises the sequence (a).

17. The antisense oligonucleotide or the pharmaceutically acceptable salt or hydrate thereof according to any one of claims 1 to 16, wherein the antisense oligonucleotide consists of 20 nucleotides.

18. The antisense oligonucleotide or the pharmaceutically acceptable salt or hydrate thereof according to any one of claims 1 to 17, wherein the wing regions comprise a 2'-OMe (2'-O-CH₃) group and/or a 2'-O-MOE (2'-O-CH₂CH₂OCH₃) group.

19. The antisense oligonucleotide or the pharmaceutically acceptable salt or hydrate thereof according to claim 17 or 18, wherein a bond between the second and third nucleosides and a bond between the fourth and fifth nucleosides from the 5' end of the 5' wing region are phosphodiester bonds, and a bond between the first and second nucleosides and a bond between the third and fourth nucleosides from the 5' end of the 3' wing region are phosphodiester bonds, while all bonds between the other nucleosides are phosphorothioate bonds.

20. The antisense oligonucleotide or the pharmaceutically acceptable salt or hydrate thereof according to claim 17 or 18, wherein a bond between the second and third nucleosides, a bond between the third and fourth nucleosides, and a bond between the fourth and fifth nucleosides from the 5' end of the 5' wing region are phosphodiester bonds, and a bond between the first and second nucleosides, a bond between the second and third nucleosides, and a bond between the third and fourth nucleosides from the 5' end of the 3' wing region are phosphodiester bonds, while all bonds between the other nucleosides are phosphorothioate bonds.

21. A pharmaceutical composition comprising the antisense oligonucleotide or the pharmaceutically acceptable salt or hydrate thereof according to any one of claims 1 to 20.

22. The pharmaceutical composition according to claim 21 for the treatment and/or prevention of infection with a virus selected from the group consisting of SARS-CoV-2, SARS-CoV-1, and MERS-CoV.

23. A method for treatment and/or prevention of infection with a virus selected from the group consisting of SARS-CoV-2, SARS-CoV-1, and MERS-CoV, which comprises administering to a subject an effective amount of the nucleic acid or the pharmaceutically acceptable salt or hydrate thereof according to any one of claims 1 to 20 or the pharmaceutical composition according to claim 21 or 22.
